(19) Europäisches Patentamt
European Patent Office
Office européen des brevets

(11) EP 2 694 469 B1

(12) # FASCICULE DE BREVET EUROPEEN

(45) Date de publication et mention de la délivrance du brevet:
**28.09.2016 Bulletin 2016/39**

(21) Numéro de dépôt: **12717879.6**

(22) Date de dépôt: **04.04.2012**

(51) Int Cl.:
***C07C 231/10*** *(2006.01)* ***C07D 231/12*** *(2006.01)*
***C07D 233/06*** *(2006.01)* ***C07D 233/58*** *(2006.01)*
***C07C 233/03*** *(2006.01)* ***C07D 241/04*** *(2006.01)*
***C07D 243/28*** *(2006.01)* ***C07D 249/02*** *(2006.01)*
***C07D 249/16*** *(2006.01)* ***C07D 251/28*** *(2006.01)*
***C07C 251/86*** *(2006.01)* ***C07D 295/185*** *(2006.01)*
***B01J 31/12*** *(2006.01)* ***C07D 249/08*** *(2006.01)*
***C07B 59/00*** *(2006.01)*

(86) Numéro de dépôt international:
**PCT/IB2012/051667**

(87) Numéro de publication internationale:
**WO 2012/137152 (11.10.2012 Gazette 2012/41)**

(54) **PROCEDE DE PREPARATION DE COMPOSES FORMAMIDES**

VERFAHREN ZUR HERSTELLUNG VON FORMAMIDVERBINDUNGEN

METHOD FOR PREPARING FORMAMIDE COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.04.2011 FR 1152888**
**16.05.2011 FR 1154246**

(43) Date de publication de la demande:
**12.02.2014 Bulletin 2014/07**

(73) Titulaire: **Commissariat à l'Énergie Atomique et aux Énergies Alternatives**
**75015 Paris (FR)**

(72) Inventeurs:
- **CANTAT, Thibault**
**F-92130 Issy Les Moulineaux (FR)**
- **GOMES, Christophe**
**F-92160 Antony (FR)**
- **JACQUET, Olivier**
**F-91400 Orsay (FR)**

(74) Mandataire: **Gevers & Orès**
**41 avenue de Friedland**
**75008 Paris (FR)**

(56) Documents cités:
**EP-A1- 0 652 202 GB-A- 783 582**

- **DATABASE WPI Week 197718 Thomson Scientific, London, GB; AN 1977-31676Y XP002661499, & JP 52 036617 A (MITSUI PETROCHEM KK) 22 mars 1977 (1977-03-22)**
- **DATABASE WPI Week 198750 Thomson Scientific, London, GB; AN 1987-352140 XP002661500, & JP 62 255456 A (IDEMITSU PETROCHEM CO) 7 novembre 1987 (1987-11-07)**
- **ANGELA DIBENEDETTO ET AL: "Reaction of silylalkylmono- and silylalkyldi-amines with carbon dioxide: evidence of formation of inter- and intra-molecular ammonium carbamates and their conversion into organic carbamates of industrial interest under carbon dioxide catalysis", GREEN CHEMISTRY, vol. 4, no. 5, 29 juillet 2002 (2002-07-29), pages 439-443, XP055009762, ISSN: 1463-9262, DOI: 10.1039/b205319p**
- **DATABASE WPI Week 198139 Thomson Scientific, London, GB; AN 1981-70575D XP002661501, & JP 56 099450 A (SAGAMI CHEM RES CENTRE) 10 août 1981 (1981-08-10)**
- **DANIELA BELLI DELL'AMICO ET AL: "Converting Carbon Dioxide into Carbamato Derivatives+", CHEMICAL REVIEWS, vol. 103, no. 10, 1 octobre 2003 (2003-10-01), pages 3857-3898, XP055009686, ISSN: 0009-2665, DOI: 10.1021/cr940266m**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**(Cont. page suivante)**

- CHRISTOPHE DAS NEVES GOMES ET AL: "A Diagonal Approach to Chemical Recycling of Carbon Dioxide: Organocatalytic Transformation for the Reductive Functionalization of CO2", ANGEWANDTE CHEMIE INTERNATIONAL EDITION, 29 septembre 2011 (2011-09-29), pages 1-6, XP55009548, ISSN: 1433-7851, DOI: 10.1002/anie.201105516

## Description

La présente invention concerne un procédé de préparation de composés formamides utilisant le dioxyde de carbone et l'utilisation de ce procédé dans la fabrication de vitamines, de produits pharmaceutiques, de colles, de fibres acryliques et cuirs synthétiques, de pesticides, et d'engrais.

Elle concerne également un procédé de fabrication de vitamines, de produits pharmaceutiques, de colles, de fibres acryliques, de cuirs synthétiques, de pesticides, et d'engrais, comprenant une étape de préparation de composés formamides par le procédé selon l'invention.

L'utilisation du $CO_2$ valorisable comme source de carbone pour la production de consommables chimiques est un défi de premier plan pour diminuer son accumulation atmosphérique mais également pour contrôler notre dépendance en combustibles fossiles.

Le plus grand défi auquel font face les scientifiques et industriels est de recycler le $CO_2$, c'est-à-dire de développer des réactions permettant de produire des composés chimiques comme, par exemple, des combustibles, des polymères plastiques, des médicaments, des détergents, des molécules à hauts tonnages, traditionnellement obtenus par des méthodes pétrochimiques. La difficulté technique réside dans la mise au point de réactions chimiques qui permettent de fonctionnaliser le $CO_2$ tout en réduisant le centre carboné (i.e. en substituant les liaisons C-O du $CO_2$ par des liaisons C-H ou C-C).

Compte tenu de la grande stabilité thermodynamique du dioxyde de carbone, sa conversion vers de nouveaux consommables chimiques fait nécessairement appel à une source énergétique extérieure de manière à favoriser le bilan thermodynamique de la transformation chimique représenté en Figure 1.

Aujourd'hui, l'ensemble des efforts de la communauté scientifique se focalise sur l'emploi d'électricité ou de lumière pour réaliser l'électroréduction ou la photoréduction du $CO_2$ en acide formique, méthanal, méthanol et méthane (Morris, A. J., Meyer, G. J., Fujita, E., Accounts Chem Res 2009, 42, 1983). De fait, ce domaine de recherche fait l'objet d'une compétition internationale intense.

Un article récent décrit que l'utilisation de composés silanes permet de réduire le $CO_2$ en conditions organocatalytiques (Riduan, S. N., Zhang, Y. G., Ying, J. Y., Angewandte Chemie-International Edition 2009, 48, 3322). Dans ce cas, le composé silane est l'espèce réactive haute en énergie, et l'utilisation du catalyseur favorise le bilan cinétique. Les auteurs décrivent la formation de produits silylés de types formyl (SiOCHO), acétal ($SiOCH_2OSi$) et méthoxy ($SiOCH_3$). Si cette stratégie est justifiée par l'importance des utilisations des produits de réduction du $CO_2$ dans l'industrie chimique (HCOOH, $H_2CO$, $CH_3OH$), il convient néanmoins de noter que ces molécules sont actuellement utilisées sur une échelle qui reste très faible par rapport à la quantité de $CO_2$ valorisable disponible. En d'autres termes, si ces molécules étaient produites exclusivement à partir de $CO_2$, elles ne permettraient de valoriser, compte tenu du marché actuel, que 3,4% du $CO_2$ valorisable produit chaque année (2,5 Gt/an) (Panorama des voies de valorisation du $CO_2$, ADEME, Juin 2010, http://www2.ademe.fr/servlet/getDoc?cid=96&m=3&id=72052&p1=30&ref=12441).

Ainsi, il est nécessaire de chercher à diversifier la nature et le nombre de consommables chimiques que l'on peut obtenir à partir du $CO_2$.

Une autre stratégie de conversion du $CO_2$ vers de nouveaux consommables chimiques, consiste à utiliser un partenaire chimique réactif (haut en énergie) pour favoriser le bilan thermodynamique de la transformation chimique de $CO_2$. Cette stratégie est encore très peu représentée dans le paysage scientifique mais elle permettra, à terme, d'ouvrir considérablement l'offre de molécules disponibles à partir de $CO_2$. Le seul procédé industriel reposant sur cette approche est la synthèse de l'urée obtenue par condensation d'ammoniac sur $CO_2$ comme indiqué dans l'équation 1 ci-dessous (Sakakura, T., Choi, J. C., Yasuda, H., Chem Rev 2007, 107, 2365*)*.

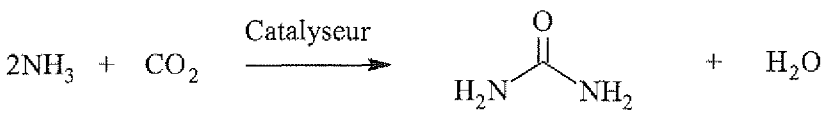


(équation 1)

Selon le même principe, la synthèse de polycarbonates par copolymérisation $CO_2$/époxydes est en voie d'industrialisation comme indiqué dans l'équation 2 ci-dessous (Panorama des voies de valorisation du $CO_2$, ADEME, Juin 2010, http://www2.ademe.fr/servlet/getDoc?cid=96&m=3&id=72052&p1=30&ref=12441).

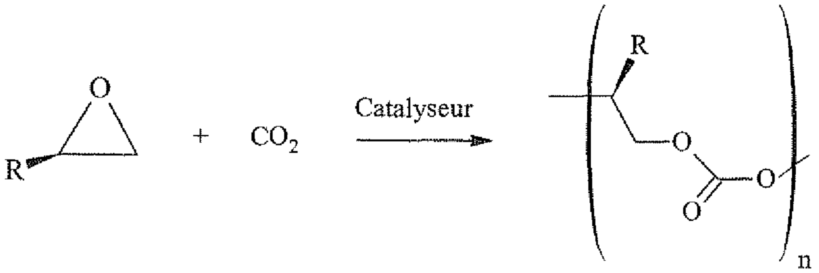


(équation 2)

Dans ces deux synthèses (équations 1 et 2), il n'y a aucune réduction formelle du centre carboné de $CO_2$.

Toujours dans le but d'obtenir de nouveaux consommables chimiques, on peut envisager de convertir le $CO_2$ en composés formamides. Les composés formamides sont une classe de composés chimiques importants dans l'industrie chimique où ils sont couramment utilisés comme solvants, réactifs et précurseurs de matériaux plastiques (The Amide Linkage: Structural Significance in Chemistry, Biochemistry, and Materials Science, Greenberg, A. B., C. M., Liebman, J. F.; Wiley-Interscience: Hoboken, New Jersey, 2002). Les composés formamides (de formule générale $R^1R^2NCHO$) sont généralement synthétisés par condensation des amines avec l'acide formique.

Le N,N-diméthylméthanamide (appelé également diméthylformamide), qui est le composé formamide le plus couramment utilisé étant donné qu'il sert de solvant polaire, est produit industriellement par réaction entre la diméthylamine et du monoxyde de carbone en conditions catalytiques (The Amide Linkage: Structural Significance in Chemistry, Biochemistry, and Materials Science, Greenberg, A. B., C. M., Liebman, J. F.; Wiley-Interscience: Hoboken, New Jersey, 2002).

Les composés formamides peuvent également être obtenus à partir du dioxyde de carbone, à la place du monoxyde de carbone qui est toxique. En effet, cette voie alternative repose sur la synthèse des composés formamides par réaction entre du dioxyde de carbone, du dihydrogène et une amine en présence de complexes des métaux de transition comme catalyseur (Schreiner, S., Yu, J. Y.; Vaska, L., Inorganica Chimica Acta 1988, 147, 139 ; Schreiner, S., Yu, J. Y., Vaska, L. Journal of the Chemical Society-Chemical Communications 1988, 602 ; Vaska, L., Schreiner, S., Felty, R. A., Yu, J. Y., Journal of Molecular Catalysis 1989, 52, L11 ; Yu, J. Y., Schreiner, S., Vaska, L. Inorganica Chimica Acta 1990, 170, 145 ; Jessop, P. G., Hsiao, Y., Ikariya, T., Noyori, R., Journal of the American Chemical Society 1994, 116, 8851 ; Jessop, P. G., Hsiao, Y., Ikariya, T., Noyori, R. Journal of the American Chemical Society 1996, 118, 344 ; Munshi, P., Heldebrant, D. J., McKoon, E. P., Kelly, P. A., Tai, C. C., Jessop, P. G., Tetrahedron Letters 2003, 44, 2725) ; Schmid, L., Rohr, M., Baiker, A., Chemical Communications 1999, 2303 ; Federsel, C., Boddien, A., Jackstell, R., Jennerjahn, R., Dyson, P. J., Scopelliti, R., Laurenczy, G., Beller, M., Angew. Chem. Int. Ed. 2010, 49, 9777 ; Liu, J., Guo, C., Zhang, Z., Jiang, T., Liu, H., Song, J., Fan, H., Han, B., Chemical Communications 2010, 46, 5770. Kiso Y. et al. (JP 52036617) décrit un procédé de production de composés formamides par réaction entre du dioxyde de carbone, du dihydrogène et une amine primaire ou secondaire en présence de complexes des ruthénium comme catalyseur.

Fujiwara Y. et al. (JP 62255456) décrit un procédé de production de N,N-diéthylformamide à partir du dioxyde de carbone, de la diéthylamine et de l'éthylène glycol mono-méthyl éther en présence de $PdCl_2(CH_3CN)_2$ comme catalyseur.

Ikariya, T. et al. (EP 652 202) décrit un procédé de production de composés formamides où le dioxyde de carbone supercritique réagit avec une amine primaire ou secondaire et de l'hydrogène en présence d'un catalyseur à base de métaux de transition tels que le rhodium, le palladium, le ruthénium, l'iridium ou le platine. Cette voie présente de nombreux inconvénients, en particulier :

- o le choix de l'amine de départ est très limité : diméthylamine, diéthylamine, pipéridine et aniline (Schreiner, S., Yu, J. Y.; Vaska, L., Inorganica Chimica Acta 1988, 147, 139 ; Schreiner, S., Yu, J. Y., Vaska, L. Journal of the Chemical Society-Chemical Communications 1988, 602 ; Vaska, L., Schreiner, S., Felty, R. A., Yu, J. Y., Journal of Molecular Catalysis 1989, 52, L11 ; Yu, J. Y., Schreiner, S., Vaska, L. Inorganica Chimica Acta 1990, 170, 145 ; Jessop, P. G., Hsiao, Y., Ikariya, T., Noyori, R., Journal of the American Chemical Society 1994, 116, 8851 ; Jessop, P. G., Hsiao, Y., Ikariya, T., Noyori, R. Journal of the American Chemical Society 1996, 118, 344 ; Munshi, P., Heldebrant, D. J., McKoon, E. P., Kelly, P. A., Tai, C. C., Jessop, P. G., Tetrahedron Letters 2003, 44, 2725) ; Schmid, L., Rohr, M., Baiker, A., Chemical Communications 1999, 2303 ; Federsel, C., Boddien, A., Jackstell, R., Jennerjahn, R., Dyson, P. J., Scopelliti, R., Laurenczy, G., Beller, M., Angew. Chem. Int. Ed. 2010, 49, 9777 ; Liu, J., Guo, C., Zhang, Z., Jiang, T., Liu, H., Song, J., Fan, H., Han, B., Chemical Communications 2010, 46, 5770 ;
- o la pression en $CO_2$ et $H_2$ nécessaire est en général élevée : de 100 à 250 bars et à 100°C (à l'exception d'un système au platine actif à la température ambiante et sous 1 bar de pression décrit par Schreiner, S., Yu, J. Y., Vaska, L., Journal of the Chemical Society-Chemical Communications 1988, 602) ;
- o cette voie nécessite l'emploi de complexes des métaux de transition souvent coûteux (Ir, Ru, Rh, Pt, Cu, Fe) ;
- o l'utilisation d'un solvant organique est généralement nécessaire, à l'exception de quelques exemples isolés de

réactions dans du $CO_2$ supercritique (Jessop, P. G., Hsiao, Y., Ikariya, T., Noyori, R., Journal of the American Chemical Society 1994, 116, 8851 ; Jessop, P. G., Hsiao, Y.; Ikariya, T., Noyori, R., Journal of the American Chemical Society 1996, 118, 344 ; Krocher, O., Koppel, R. A., Baiker, A., High Pressure Chemical Engineering 1996, 12, 91 ; Kayaki, Y., Suzuki, T., Ikariya, T. Chemistry Letters 2001, 1016 ; Liu, F. C., Abrams, M. B., Baker, R. T., Tumas, W., Chemical Communications 2001, 433 ; Kayaki, Y., Shimokawatoko, Y., Ikariya, T., Advanced Synthesis & Catalysis 2003, 345, 175), et d'un exemple en liquide ionique (Liu, F. C., Abrams, M. B., Baker, R. T., Tumas, W., Chemical Communication,s 2001, 433), et d'un exemple sans solvant (Krocher, O., Koppel, R. A., Baiker, A., Chemical Communications 1997, 453) ;

o l'ajout d'additifs (des bases carbonées, oxygénées ou azotées) est nécessaire pour accélérer la réaction ou améliorer les rendements et sélectivités (Munshi, P., Heldebrant, D. J., McKoon, E. P., Kelly, P. A.; Tai, C. C., Jessop, P. G. Tetrahedron Letters 2003, 44, 2725).

Dans le cadre de la synthèse des composés formamides en utilisant le dioxyde de carbone, le défi technique à relever est de coupler la fonctionnalisation du dioxyde de carbone à une étape de réduction chimique. Pour maximiser le rendement énergétique d'une telle transformation, il est nécessaire de développer des réactions avec un nombre limité d'étapes (idéalement une seule) et catalysées, pour éviter les pertes énergétiques d'ordre cinétique.

Par ailleurs, les composés formamides marqués, incorporant des radioisotopes et/ou isotopes stables, présente un intérêt particulier dans de nombreux domaines comme, par exemple, dans les sciences du vivant (étude/élucidation de mécanismes enzymatiques, de mécanismes biosynthétiques, en biochimie, etc.), les sciences de l'environnement (traçage de déchets, etc.), la recherche (étude/élucidation de mécanismes réactionnels) ou encore la recherche et le développement de nouveaux produits pharmaceutiques et thérapeutiques. Ainsi, développer une synthèse pour la préparation de composés formamides marqués répondant aux exigences indiquées ci-dessus, peut répondre à un besoin réel.

Il existe donc un réel besoin d'un procédé pour préparer les composés formamides par transformation du $CO_2$, palliant les inconvénients de l'art antérieur, ledit procédé permettant de coupler la fonctionnalisation du dioxyde de carbone à une étape de réduction chimique.

En particulier, il existe un réel besoin d'un procédé qui permette d'obtenir en une seule étape et avec une excellente sélectivité les composés formamides, à partir du $CO_2$ et d'amines, en conditions catalytiques et en présence d'un composé assurant la réduction de $CO_2$.

Il existe, en outre, un réel besoin de disposer d'un procédé qui permette d'obtenir, en une seule étape et avec une excellente sélectivité, les composés formamides marqués incorporant des radioisotopes et/ou isotopes stables, à partir de réactifs marqués comme par exemple le $CO_2$ marqué et/ou les amines marquées, en conditions catalytiques et en présence d'un composé assurant la réduction de $CO_2$.

La présente invention a précisément pour but de répondre à ces besoins en fournissant un procédé de préparation de composés formamides de formule (I) :

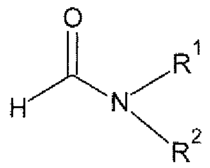

(I)

dans laquelle

- $R^1$ et $R^2$, représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, une aldimine de formule $-N=CHR^6$, une cétimine de formule $-N=CR^6R^7$, lesdits groupes alkyle, alcényle, alcynyle, aryle, hétéroaryle, hétérocycle, silylé, siloxy et amino étant éventuellement substitués, ou
- $R^1$ et $R^2$, pris ensemble avec l'atome d'azote auquel ils sont liés forment un hétérocycle éventuellement substitué, ou
- $R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$,

  et
- $R^6$ et $R^7$, représentant, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle, alcynyle, aryle, hétéroaryle, hétérocycle, silylé, siloxy et amino étant éventuellement substitués,

caractérisé en ce que l'on fait réagir une amine de formule (II) dans laquelle $R^1$ et $R^2$ sont tels que définis ci-dessus,

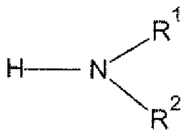

(II)

avec du $CO_2$, en présence d'un catalyseur et d'un composé silane de formule (III)

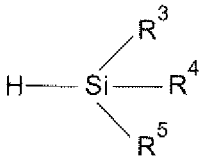

(III)

dans laquelle

- $R^3$, $R^4$ et $R^5$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^5$ est tel que défini ci-dessus et $R^3$ et $R^4$, pris ensemble avec l'atome de silicium auquel ils sont liés forment un hétérocycle silylé éventuellement substitué.

Le procédé de l'invention a l'avantage de permettre de convertir le $CO_2$ en composés formamides avec un grand choix d'amines de formule (II) (amines primaires, secondaires, aromatiques, aliphatiques, etc.). Dans ce procédé, lesdites amines servent essentiellement à fonctionnaliser le $CO_2$, et les composés silanes de formule (III) assurent la réduction de $CO_2$, en conditions catalytiques.

Les composés formamides sont ainsi obtenus avec un excellent rendement (de l'ordre de 60 à 100%, par exemple), et une excellente sélectivité (100% de composés formamides isolés).

Dans le cadre de la présente invention, le rendement est calculé par rapport à la quantité d'amine de formule (II) introduite initialement, sur la base de la quantité de formamide isolé:

$$\text{Rendement} = n(\text{amide})/(n(\text{amide})+n(\text{amine}))$$

n étant la quantité de matière

Dans le cadre de la présente invention, la sélectivité se rapporte à la nature des produits formés à partir de l'amine de formule (II). En effet, les composés formamides de formule (I) étant les seuls produits azotés formés au cours du procédé de l'invention, la sélectivité est totale.

On entend par « alkyle », au sens de la présente invention, un radical carboné linéaire, ramifié ou cyclique, saturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, heptyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle. Comme alcényles cycliques, on peut citer par exemple le cyclopentényle, le cyclohexényle. On peut citer, par exemple, les radicaux éthylényle, propylényle, butényle, pentényle, hexényle, acétylényle, propynyle, butynyle, pentynyle, hexynyle et leurs isomères ramifiés. Les groupes alkyles, alcényles et alcynyles, peuvent être éventuellement substitués par un ou plusieurs groupes hydroxyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs atomes d'halogène choisis parmi les

atomes de fluor, chlore, brome et iode, un ou plusieurs groupes nitro ($-NO_2$), un ou plusieurs groupes nitrile (-CN), un ou plusieurs groupes alkyle, un ou plusieurs groupes aryle, avec les groupes alkoxy, alkyle et aryle tels que définis dans le cadre de la présente invention.

Le terme « hétéroaryle » désigne de manière générale un substituant aromatique mono- ou polycyclique comportant de 5 à 10 membres dont au moins 2 atomes de carbone, et au moins un hétéroatome choisi parmi l'azote, l'oxygène ou le soufre. Le groupe hétéroaryle peut être mono- ou poly- cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, triazolyle, pyrazolyle, oxazolyle, isoxazolyle, benzoxazolyle, thiazolyle, benzothiazolyle, isothiazolyle, pyridazinyle, pyrimidinyle, pyrazinyle, triazinyle, cinnolinyle, phtalazinyle, quinazolinyle, 1,1-diphénylhydrazinyle, 1,2-diphénylhydrazinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, tel que défini ci-dessus, lié par un atome d'oxygène (-O-alkyle).

Le terme « hétérocycle » désigne de manière générale un substituant mono- ou polycyclique, comportant de 5 à 10 membres, saturé ou instauré, contenant de 1 à 4 hétéroatomes choisis indépendamment l'un de l'autre, parmi l'azote, l'oxygène et le soufre. A titre indicatif, on peut citer les substituants morpholinyle, pipéridinyle, pipérazinyle, pyrrolidinyle, imidazolyle, imidazolidinyle, imidazolinyle, pyrazolidinyle, tétrahydrofuranyle, tétrahydropyranyle, tétrahydropyrimidinyle, triazolyle, pyrazolyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome ou iode.

Par groupe « silylé », on entend un groupe de formule $[-Si(X)_3]$ dans lequel chaque X, indépendamment l'un de l'autre, est choisi parmi un atome d'hydrogène, un ou plusieurs atomes d'halogène choisis parmi les atomes de fluor, chlore, brome ou iode, un ou plusieurs groupes alkyle, un ou plusieurs groupes alkoxy, un ou plusieurs groupes aryle, un ou plusieurs groupes siloxy, avec les groupes alkyle, alkoxy, aryle et siloxy tels que définis dans le cadre de la présente invention.

Par groupe « siloxy », on entend un groupe silylé, tel que défini ci-dessus, lié par un atome d'oxygène ($-O-Si(X)_3$).

Au sens de l'invention, par « hétérocycle silylé », on entend un substituant mono- ou polycyclique, comportant de 5 à 15 membres, saturé ou instauré, contenant au moins un atome de silicium et éventuellement au moins un autre hétéroatome choisi parmi l'azote, l'oxygène ou le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode ; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiène, suivant les formules ci-dessous.

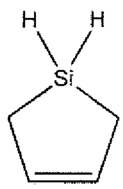

1-silacyclo-3-pentène

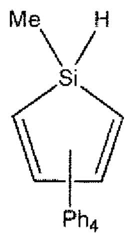

1-méthyl-1-hydrido-2,3,4,5-tétraphényl-1-silacyclopentadiène

On peut encore citer, par exemple, le méthyl siloxane, le 1-phényl-1-silacyclohexane, le 1-sila-bicyclo[2.2.1] heptane, le 1-méthyl-1-silacyclopentane, le 9,9-dihydro-5-silafluorène répondant aux formules ci-dessous.

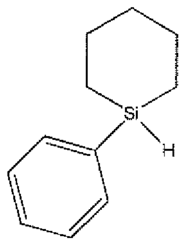

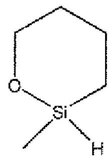

methyl siloxane

1-phenyl-1-silacyclohexane

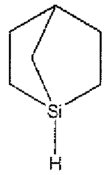

1-sila-bicyclo[2.2.1]heptane

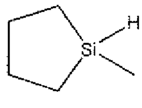

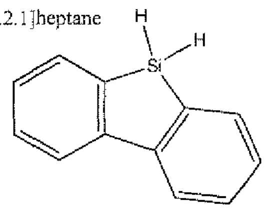

1-methyl-1-silacyclopentane

9,9-dihydro-5-silafluorene

Par groupe « amino », on entend un groupe de formule -$NR^6R^7$, dans laquelle : $R^6$ et $R^7$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention ; ou
$R^6$ et $R^7$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro (-$NO_2$) ; un ou plusieurs groupes nitrile (-CN) ; un ou plusieurs groupes aryle ; avec les groupes alkyle, alkoxy et aryle tels que définis dans le cadre de la présente invention.

Lorsque $R^1$ et $R^2$, forment avec l'atome d'azote auquel ils sont liés, une double liaison carbone-azote (N=C) pour conduire à une aldimine de formule -$N{=}CHR^6$ ou à une cétimine de formule -$N{=}CR^6R^7$, et que $R^6$ et $R^7$, représentent, indépendamment l'un de l'autre, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle, alcynyle, aryle, hétéroaryle, hétérocycle, silylé, siloxy et amino tels que définis dans le cadre de la présente invention, peuvent être éventuellement substitués par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro (-$NO_2$), un ou plusieurs groupes nitrile (-CN), un ou plusieurs groupes alkyle, avec les groupes alkyle, alkoxy et aryle tels que définis dans le cadre de la présente invention.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur.

Selon une variante préférée de l'invention, dans l'amine de formule (II), $R^1$ et $R^2$, représentent, indépendamment l'un de l'autre, un atome d'hydrogène, un groupe alkyle, un groupe aryle, un groupe hétéroaryle, un groupe amino, lesdits groupes alkyle, amino, aryle et hétéroaryle étant éventuellement substitués, ou
$R^1$ et $R^2$, pris ensemble avec l'atome d'azote auquel ils sont liés forment un hétérocycle éventuellement substitué ; ou
$R^1$ et $R^2$, forment avec l'atome d'azote auquel ils sont liés, une double liaison carbone-azote (N=C) pour conduire à une aldimine de formule -$N{=}CHR^6$ ou à une cétimine de formule -$N{=}CR^6R^7$, dans laquelle $R^6$ et $R^7$, représentent, indépendamment l'un de l'autre, un atome d'hydrogène, un groupe alkyle, un groupe aryle, un groupe hétéroaryle, un hétérocycle, lesdits groupes alkyle, aryle, hétéroaryle et hétérocycle étant éventuellement substitués.

De préférence, dans l'amine de formule (II), $R^1$ et $R^2$, représentent, indépendamment l'un de l'autre, un atome

d'hydrogène ; un groupe alkyle choisi parmi les groupes méthyle, éthyle, propyle, butyle, pentyle, hexyle, heptyle, ou leurs isomères ramifiés, cyclohexyle ; un groupe aryle choisi parmi le benzyle ou le phényle ; un groupe hétéroaryle choisi parmi l'imidazolyle ou le benzimidazolyle ; un groupe amino de formule $-NR^6R^7$, dans laquelle $R^6$ et $R^7$ représentent, un groupe aryle choisi parmi le benzyle ou le phényle, ledit groupe aryle étant éventuellement substitué ; ou $R^1$ et $R^2$, pris ensemble avec l'atome d'azote auquel ils sont liés forment un hétérocycle comportant de 5 à 6 membres choisi parmi la morpholine ; la pipéridine ; la pipérazine ; la pyrrolidine; l'oxazolidine ; ou l'isoxazolidine ; l'imidazole, en particulier, le 1H-imidazole ; la tétrahydropyrimidine, en particulier, la 1,4,5,6-tétrahydropyrimidine ; triazolyle, pyrazolyle, ou

$R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$ dans laquelle $R^6$ et $R^7$ représentent, indépendamment l'un de l'autre, un groupe aryle choisi parmi le benzyle ou le phényle, ledit groupe alkyle, alkoxy et aryle étant éventuellement substitué.

Selon une autre variante préférée de l'invention, dans le composé silane de formule (III), $R^3$, $R^4$ et $R^5$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

De préférence, dans le composé silane de formule (III), $R^3$, $R^4$ et $R^5$ représentent indépendamment l'un de l'autre,

- un atome d'hydrogène ;
- un groupe alkyle choisi parmi les groupes méthyle, éthyle, propyle, butyle, pentyle, hexyle, heptyle ou leurs isomères ramifiés ;
- un groupe alkoxy dont le groupe alkyle est choisi parmi les groupes méthyle, éthyle, propyle, butyle, pentyle, hexyle, heptyle ou leurs isomères ramifiés ;
- un groupe aryle choisi parmi les groupes benzyle ou phényle ;
- un groupe silylé de formule $[-Si(X)_3]$ dans lequel chaque X, indépendamment l'un de l'autre, est choisi parmi un atome d'hydrogène, un ou plusieurs atomes d'halogène choisis parmi les atomes chlore, brome, ou iode, un ou plusieurs groupes alkyle choisi parmi les groupes méthyle, éthyle, propyle, butyle, pentyle, hexyle, heptyle ou leurs isomères ramifiés, un ou plusieurs groupes alkoxy dont le groupe alkyle est choisi parmi les groupes méthyle, éthyle, propyle, butyle, pentyle, hexyle, heptyle ou leurs isomères ramifiés, un ou plusieurs groupes siloxy dont le groupe $-Si(X)_3$ est tel que décrit dans ce mode de réalisation.

Les catalyseurs peuvent être choisis parmi les catalyseurs organiques ou les catalyseurs métalliques choisis parmi les sels ou complexes métalliques. Les catalyseurs organiques présentent l'avantage de permettre de s'affranchir des problèmes de toxicité généralement observés pour les catalyseurs métalliques ainsi que des problèmes de coûts associés à l'utilisation de métaux précieux. Dans le procédé de l'invention, le catalyseur est, de préférence, organique.

Les catalyseurs organiques sont, en général, des bases organiques, choisies parmi :

- les bases azotées, comme par exemple, les amines secondaires ou tertiaires choisies parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ;
- les bases phosphorées, comme par exemple, les alkyles et aryle phosphines choisi parmi le triphénylphosphine, le 2,2'-bis(diphénylphosphino)-1,1'-binaphthyle (BINAP), le triisopropylphosphine ; les alkyle et aryle phosphonates choisis parmi le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi le di-n-butylphosphate (DBP), le tris-(2-éthylhexyl)-phosphate, le triéthyl phosphate ;
- les bases carbonées pour lesquelles la protonation a lieu sur un atome de carbone, comme par exemple, un carbène N-hétérocyclique comme un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

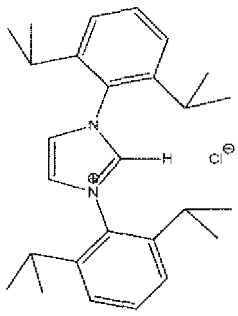 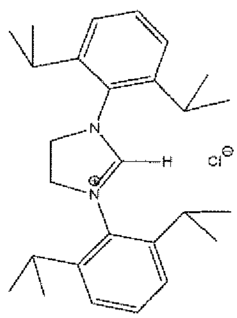 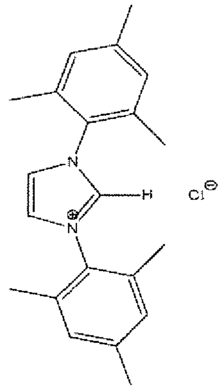 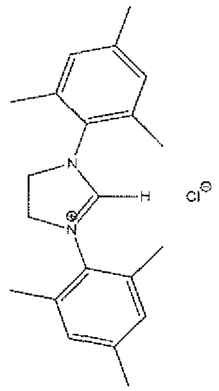

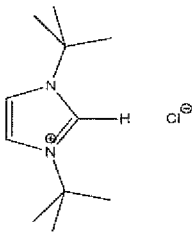 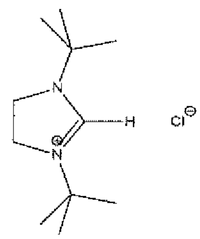 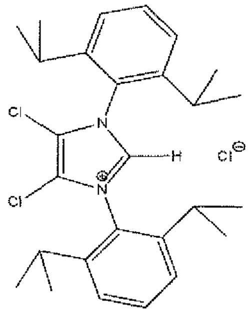

ou

- les bases oxygénées, comme par exemple le peroxyde d'hydrogène ; le peroxyde de benzoyle ; un alcoolate choisi parmi le méthanolate, l'éthanolate, le propanolate, le butanolate, le pentanolate, l'hexanolate, de sodium ou de potassium.

Le catalyseur organique est avantageusement :

- une amine secondaire ou tertiaire choisie parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou
- un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

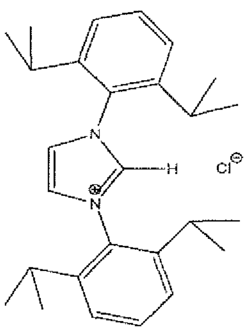 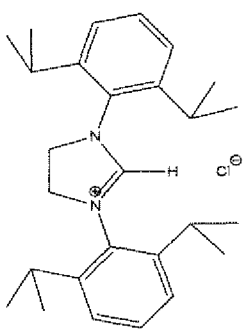 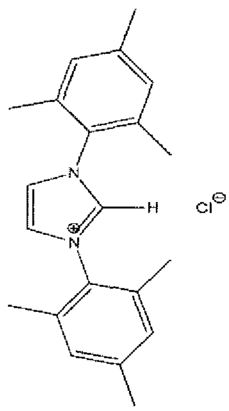 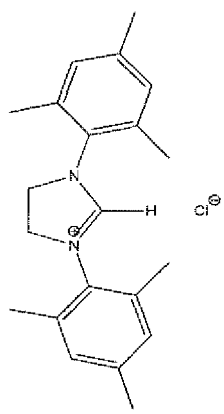

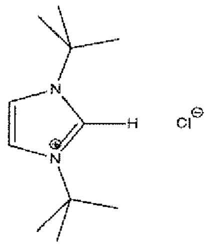 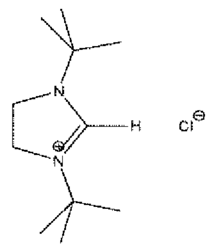 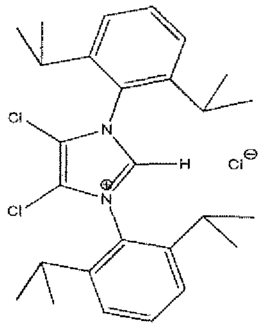

Selon une variante préférée de l'invention, le catalyseur organique est choisi parmi le triazabicyclodécène (TBD), le N-méthyltriazabicyclodécène (MeTBD), le 1,8-diazabicyclo[5.4.0]undéc-7-ène (DBU) ; ou un carbène N-hétérocyclique tel qu'un carbène issu d'un sel d'imidazolium tel que le chlorure de 1,3-bis(2,6-diisopropylphenyl)-1H-imidazol-3-ium (carbène A), le chlorure de 1,3-bis(2,6-diisopropylphenyl)-4,5-dihydro-1H-imidazol-3-ium (carbène C), le chlorure de 1,3-di-tert-butyl-1H-imidazol-3-ium, le chlorure de 1,3-di-tert-butyl-4,5-dihydro-1H-imidazol-3-ium (carbène F), le chlorure de 1,3-bis(2,4,6-triméthylphenyl)-1H-imidazol-3-ium (carbène B), le chlorure de 1,3-bis(2,4,6-triméthylphenyl)-4,5-dihydro-1H-imidazol-3-ium (carbène D), le chlorure de 4,5-dichloro-1,3-bis(2,6-diisopropylphenyl)-1H-imidazol-3-ium (carbène E).

Lorsque le catalyseur est un catalyseur métallique, il peut être choisi parmi les sels ou complexes de :

- métaux choisis parmi le bore, le silicium, l'aluminium, le gallium, l'étain, l'indium, par exemple, $Al(OiPr)_3$, $SnCl_2$, $InBr_3$ ;
- métaux alcalins choisis parmi le sodium, le potassium, par exemple, $Na_2CO_3$, $K_2CO_3$, $Cs_2CO_3$ ;
- métaux alcalinoterreux choisis le magnésium, le calcium, par exemple, $MgSO_4$, $Ca(BH_4)_2$ ;
- métaux de transition choisis parmi le nickel, le fer, le cobalt, le zinc, le cuivre, le rhodium, le ruthénium, le platine, le palladium, l'iridium, par exemple, $Fe(BH_4)_2.6H_2O$, CuCl, $ZnEt_2$ ;
- terres rares choisis parmi le lanthane, le cérium, le praséodyme, le néodyme, par exemple, $La(OTf)_3$, $CeCl_3$.

Par complexe métallique il est entendu un composé organométallique ou inorganique de coordination dans lequel un ion métallique est lié à un ligand organique ou inorganique. Un complexe organométallique ou inorganique peut être obtenu par mélange d'un sel métallique avec un ligand, celui-ci se liant au métal par des atomes de phosphore, de carbone, d'azote, d'oxygène, d'hydrogène ou de silicium, par exemple. Comme ligand organique ou inorganique, on peut citer par exemple, le tris[2-diphénylephosphino)éthyle]phosphine ($PP_3$), le carbène A, la tricyclohexylphosphine.

Le catalyseur métallique est, avantageusement, choisi parmi les sels ou complexes de métaux de transition choisis parmi le nickel, le fer, le cobalt, le zinc, le cuivre, le rhodium, le ruthénium, le platine, le palladium, l'iridium, par exemple, $Fe(BH_4)_2.6H_2O$, CuCl, $ZnEt_2$.

Sans vouloir être lié par la théorie, dans le cas des catalyseurs métalliques, le pré-catalyseur réagit avec l'amine $R^1R^2NH$ pour former un complexe amidure, capable de réagir avec une molécule de $CO_2$. Le carbamate métallique résultant est finalement réduit par le composé silane, formant une molécule du composé formamide ainsi qu'un complexe silanolate. Ce dernier est capable de régénérer le catalyseur amidure par une métathèse sigma telle que représentée en Figure 2. Dans le cas des catalyseurs organiques, en particulier les bases organiques, selon la nature des pré-catalyseurs, trois mécanismes différents peuvent avoir lieu :

- *Mécanisme par activation du composé silane*
  Ce mécanisme est suivi par les catalyseurs nucléophiles, typiquement les carbènes N-hétérocycliques. Il est représenté en Figure 3 avec l'exemple d'un carbène N-hétérocyclique ;
- *Mécanisme par activation du carbamate*
  Ce mécanisme est suivi par les catalyseurs basiques et peu nucléophiles, de type TBD, DBU, Me-TBD notamment. Il est représenté en Figure 4 avec l'exemple de la TBD ;
- *Mécanisme par trans-formylation*
  Ce mécanisme est suivi par les catalyseurs basiques possédant une liaison N-H pouvant être formylée en présence de $CO_2$ et d'un composé silane $R^3R^4R^5SiH$. La fonction formyle (CHO) est ensuite transférée au substrat amine $R^1R^2NH$. Ce cycle catalytique, par exemple avec la TBD, a été observé en compétition avec le cycle catalytique représenté dans la Figure 4. Ce mécanisme par trans-formylation est représenté en Figure 5 avec l'exemple de la TBD.

Les catalyseurs peuvent, le cas échéant, être immobilisés sur des supports hétérogènes afin d'assurer une

séparation facile dudit catalyseur et/ou son recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; l'alumine ; ou le chlorure de magnésium ($MgCl_2$).

Dans le procédé selon l'invention, la réaction peut se produire sous une pression de $CO_2$, en faisant barboter du $CO_2$ dans le milieu réactionnel ou sous une atmosphère sèche contenant du $CO_2$ (air ambiant séché comprenant, par exemple environ 78% en volume d'azote, 21% en volume d'oxygène, et d'environ de 0,2 à 0,04% en volume de dioxyde de carbone). La réaction peut également se produire en utilisant du $CO_2$ supercritique.

De préférence, la réaction se produit sous une pression de $CO_2$.

La pression du $CO_2$, peut alors être comprise entre 1 et 50 bars, de préférence entre 1 et 30, plus préférentiellement entre 1 et 10 bars, bornes incluses.

La température de la réaction peut être comprise entre 25 et 150°C, de préférence entre 50 et 125°C, plus préférentiellement entre 70 et 100°C, bornes incluses.

La durée de la réaction dépend du taux de conversion de l'amine de formule (II). La réaction est avantageusement maintenue jusqu'à la conversion totale de l'amine de formule (II). La réaction est effectuée pendant une durée de 5 minutes à 72 heures, avantageusement de 15 minutes à 48 heures, de préférence de 1 à 48 heures, bornes incluses.

Le procédé de l'invention, en particulier la réaction entre les différents réactifs, peut avoir lieu dans un ou un mélange d'au moins deux solvant(s) choisi(s) parmi :

- les éthers, de préférence, l'éther diéthylique, ou le tetrahydrofuran (THF) ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

Selon une variante préférée de l'invention, il n'est pas nécessaire d'ajouter un solvant supplémentaire. Dans ce cas l'amine de formule (II) est le solvant. Ainsi, outre son rôle de fonctionnalisation du $CO_2$, l'amine sert de solvant.

Le rapport molaire entre le composé silane de formule (III) et l'amine de formule (II) est de 0,5 à 5, de préférence de 1 à 3, bornes incluses.

La quantité de catalyseur est de 0,001 à 1 équivalent molaire, de préférence de 0,01 à 1 équivalent molaire, plus préférentiellement de 0,01 à 0,9 équivalent molaire, encore plus préférentiellement de 0,01 à 0,5 équivalent molaire, bornes incluses, par rapport à l'amine de formule (II).

L'invention concerne également, le procédé de préparation de composés de composés formamides marqués de formule (I') :

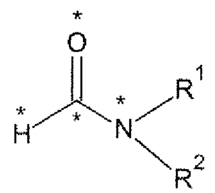

(I')

dans laquelle

- ■ $R_1$ et $R_2$ sont tels que définis précédemment,
- ■ $H^*$ représente un atome d'hydrogène ($^1H$), le deutérium ($^2H$) ou le tritium ($^3H$),
- ■ $O^*$ représente un atome d'oxygène ($^{16}O$), un isotope $^{17}O$ ou $^{18}O$,
- ■ $C^*$ représente un atome de carbone ($^{12}C$), un isotope $^{11}C$, $^{13}C$ ou $^{14}C$,
- ■ $N^*$ représente un atome d'azote ($^{14}N$), un isotope $^{15}N$,

caractérisé en ce que l'on fait réagir une amine de formule (II') dans laquelle $R^1$, $R^2$ et $N^*$ sont tels que définis ci-dessus,

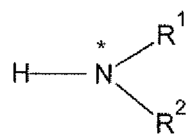

(II')

avec du $C^*O_2^*$ dans lequel $C^*$ et $O^*$ sont tels que définis ci-dessus, en présence d'un catalyseur et d'un composé silane de formule (III')

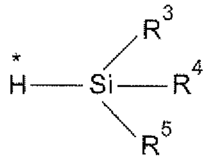

(III')

dans laquelle

- $R^3$, $R^4$, $R^5$ et $H^*$ sont tels que définis ci-dessus.

Les composés de formule (I') correspondent en fait aux composés de formule (I) comportant au moins un radiomarqueur/radiotraceur ou un isotope choisi.

Par isotopes on entend, pour un même élément, deux atomes ayant le même nombre de protons (et d'électrons) mais un nombre de neutrons différent. Ayant le même nombre d'électrons et de protons, les propriétés chimiques des isotopes d'un même élément sont presque identiques. Il peut exister, cependant, de légères variations de la vitesse d'une réaction chimique lorsqu'un des atomes d'un réactif est remplacé par un de ses isotopes. En revanche, comme le noyau ne comporte pas le même nombre de neutrons, la masse des atomes varie ce qui peut rendre l'atome instable : c'est pourquoi ils peuvent être radioactifs. Il s'agit alors de radioisotopes. Dans le cadre de l'invention, le terme « isotopes » peut également englober les « radioisotopes ».

Le radiomarquage est le fait d'associer à une molécule ou un composé donnés un isotope qui permettra de suivre l'évolution ou/et la fixation des molécules, par exemple, dans un organe. Le radiotraceur est le ou le(s) élément(s) radioactif(s) présent(s) au sein d'une molécule pour suivre le cheminement de cette substance, par exemple, dans un organe.

Ce procédé peut ainsi permettre l'accès aux composés formamides marqués $^{11}C$, $^{13}C$, $^{14}C$, $^{15}N$, $^{2}H$ (D), $^{3}H$ (T), $^{17}O$ et $^{18}O$.

L'utilisation de molécules à des fins de traçage, de métabolisation, d'imagerie, etc., est détaillée dans la littérature (U. Pleiss, R. Voges, "Synthesis and Applications of Isotopically Labelled Compounds, Volume 7". Wiley-VCH, 2001 ; R. Voges, J. R. Heys, T. Moenius, "Preparation of Compounds Labeled with Tritium and Carbon-14". Wiley-VCH: Chippenham (UK), 2009). La possibilité de former les composés formamides marqués peut être assurée par la disponibilité des réactifs marqués correspondant, par exemple, par :

- les amines $R^1R^2NH$ enrichies en $^{15}N$ sont accessibles à partir du chlorure d'ammonium enrichi $^{15}N$ : $[^{15}NH_4][Cl]$ (Yong-Joo Kim, Max P. Bernstein, Angela S. Galiano Roth, Floyd E. Romesberg, Paul G. Williard, David J. Fuller, Aidan T. Harrison, and David B. Collum, J. Org. Chem. 1991, 56, p. 4435-4439).

- le $CO_2$ marqué $^{11}C$ ou $^{14}C$ est la source principale de $^{11}C$ et $^{14}C$ est obtenue par acidification du carbonate de baryum marqué $Ba^{14}CO_3$.( R. Voges, J. R. Heys, T. Moenius, "Préparation of Compounds Labeled with Tritium and Carbon-14". Wiley-VCH: Chippenham (UK), 2009).
- le $CO_2$ marqué $^{17}O$ ou $^{18}O$ est utilisé en chimie de synthèse pour produire des molécules marquées $^{17}O$ et $^{18}O$ (Christopher J. Dinsmore and Swati P. Mercer, Organic Letters, 2004 Vol., 6, No. 17 2885-2888 ; John T. Groves and Yoshihito Watanabe, J. Am. Chem. SOC. 1988, 110, p. 8443-8452).
- les silanes $R^3R^4R^5Si-H$ marqués $^{2}H$ (deutérium ou D) ou $^{3}H$ (tritium ou T) sont accessibles à partir du chlorosilane correspondant $R^3R^4R^5Si-Cl$ et d'hydrure de lithium (LiH) ou tétrahydruroaluminate de lithium ($LiAlH_4$), les hydrures étant disponibles tous deux en versions deutérée et tritiée (T. A. Kochina, D. V. Vrazhnov, E. N. Sinotova, V. V. Avrorin, M. Yu. Katsap, and Yu. V. Mykhov, Russian Journal of General Chemistry, Vol. 72, No. 8, 2002, p. 1222-1224 ; E. A. Shishigin, V. V. Avrorin, T. A. Kochina, and E. N. Sinotova, Russian Journal of General Chemistry, Vol. 75, No. 1, 2005, p. 152).

Si le marquage de composés formamides est possible avec les noyaux $^{11}C$, $^{13}C$, $^{14}C$, $^{15}N$, $^{2}H$ (D), $^{3}H$ (T), $^{17}O$ et $^{18}O$, l'application visant à la formation de molécules marquées $^{14}C$ peut être la plus prometteuse en termes d'impact et de demande.

Les molécules marquées $^{14}C$ ont contribué à de nombreuses avancées dans les sciences du vivant (mécanismes enzymatiques, mécanismes biosynthétiques, biochimie), les sciences de l'environnement (traçage de déchets), la recherche (élucidation de mécanismes réactionnels) ou encore le diagnostic, la recherche et le développement de

nouveaux produits pharmaceutiques et thérapeutiques. Les molécules marquées $^{14}C$ présentent, en effet, un avantage pour les études métaboliques car le $^{14}C$ est facilement détectable et quantifiable en milieu *in vitro* comme *in vivo*.

La source principale de $^{14}C$ est le $^{14}CO_2$ qui est obtenu par acidification du carbonate de baryum $Ba^{14}CO_3$. La mise au point de procédés de synthèse de molécules de base servant à l'élaboration de médicaments est donc primordiale pour produire des principes actifs marqués $^{14}C$ dont le métabolisme pourra ainsi être déterminé (R. Voges, J. R. Heys, T. Moenius, "Preparation of Compounds Labeled with Tritium and Carbon-14". Wiley-VCH: Chippenham (UK), 2009).

La contrainte majeure limitant la synthèse de molécules marquées $^{14}C$ est la nécessité d'avoir un rendement élevé en produit $^{14}C$ formé par rapport à la quantité de $^{14}CO_2$ utilisée et de reposer sur un nombre restreint d'étapes afin de limiter au maximum les coûts liés à l'utilisation de $Ba^{14}CO_3$ (U. Pleiss, R. Voges, "Synthesis and Applications of Isotopically Labelled Compounds, Volume 7". Wiley-VCH, 2001 ; R. Voges, J. R. Heys, T. Moenius, "Preparation of Compounds Labeled with Tritium and Carbon-14". Wiley-VCH: Chippenham (UK), 2009).

Le procédé selon l'invention répond à ces exigences car la pression de travail en $CO_2$ peut être faible, par exemple de 0,2 à 1 bar. En outre, le taux d'incorporation en $CO_2$ (ou rendement par rapport au $CO_2$ introduit) reste élevé, et peut, par exemple, dépasser les 95%.

Enfin, la synthèse de composés formamides marqués $^{14}C$ selon la présente invention, est une amélioration très nette par rapport aux technologies connues reposant, généralement, sur un minimum de deux étapes, dans lesquelles le $CO_2$ est d'abord réduit en acide formique pour être ensuite converti en composés formamides (J. Z. Ho and coll, Helvetica Chimica Acta, 2005, 88, p.1040). Le procédé de l'invention peut donc permettre, d'accéder aux composés formamides en une seule étape à partir du $CO_2$ avec de bons rendements et une bonne sélectivité. L'intérêt des formamides marqués $^{14}C$ pour la synthèse de molécules complexes marquées $^{14}C$ est illustrée dans les références suivantes dans le cas de principes actifs pharmaceutiques : J. Z. Ho and coll, Helvetica Chimica Acta, 2005, 88, p.1040 ; I. V. Ekhato, S. Bonacorsi Jr., J., Label Compd. Radiopharm, 2011, 54, p.202-205 ; Kenneth K. Chan, James A. Staroscik, Journal of Médicinal Chemistry, 1977, Vol. 20, No. 4, p.598.

Dans cette variante du procédé selon l'invention, lorsque la réaction se produit sous une pression de $CO_2$, la pression du $CO_2$, peut alors être comprise entre 0,2 et 50 bars, de préférence entre 0,2 et 30, plus préférentiellement entre 0,2 et 10 bars, bornes incluses.

L'invention a également pour objet l'utilisation du procédé de préparation de composés formamides de formule (I) selon l'invention, dans la fabrication de vitamines, de produits pharmaceutiques, de colles, de fibres acryliques et cuirs synthétiques, de pesticides, et d'engrais.

L'invention a, en outre, pour objet un procédé de fabrication de vitamines, de produits pharmaceutiques, de colles, de fibres acryliques, de cuirs synthétiques, de pesticides, d'engrais, caractérisé en ce qu'il comprend une étape de préparation de composés formamides de formule (I) par le procédé selon l'invention.

Comme déjà indiqué, le procédé selon l'invention conduit à la formation de composés formamides avec un excellent rendement (de l'ordre de 60 à 100%, par exemple), et une excellente sélectivité (100% de composés formamides isolés). Une simple filtration peut permettre de récupérer le catalyseur éventuellement supporté et d'éliminer les éventuels sous-produits silylés formés.

D'autres avantages et caractéristiques de la présente invention apparaîtront à la lecture des exemples ci-dessous et des Figures annexées dans lesquelles :

- la Figure 1 représente la stabilité thermodynamique du dioxyde de carbone, et la nécessité de faire appel à une source énergétique extérieure pour favoriser le bilan thermodynamique de la transformation chimique et la conversion de $CO_2$ vers de nouveaux consommables chimiques.
- la Figure 2 représente le mécanisme de transformation du $CO_2$ en présence d'un catalyseur métallique ;
- la Figure 3 représente le mécanisme de transformation du $CO_2$, par activation du composé silane, en présence d'un catalyseur nucléophile, comme un carbène N-hétérocyclique ;
- la Figure 4 représente le mécanisme de transformation du $CO_2$, par activation du carbamate, en présence d'un catalyseur basique et peu nucléophile, comme la TBD ;
- la Figure 5 représente le mécanisme de transformation du $CO_2$, par transformylation, en présence de la TBD.

EXEMPLES

EXEMPLE 1 :

Le procédé de préparation de composés formamides de formule (I), peut être effectué selon le protocole expérimental suivant.

Sous atmosphère inerte, en boîte à gants, l'amine $R^1R^2NH$ (1 équivalent molaire), le (pré-)catalyseur (de 0,001 à 1 équivalent molaire), le composé silane (1 équivalent) et le solvant sont introduits dans un tube de Schlenk qui est

ensuite scellé par un robinet J. Young. La concentration en amine et en composé silane dans le mélange réactionnel est d'environ 1M (concentration calculée sur la base du volume de solvant introduit). L'ordre d'introduction des réactifs n'a pas d'importance.

Le tube de Schlenk est ensuite mis sous pression de $CO_2$ (de 1 à 3 bar) à l'aide d'une rampe à vide puis est chauffé à une température comprise entre 25 et 100 °C jusqu'à la conversion totale de l'amine (de 5 minutes à 72 heures de réaction).

La réaction une fois terminée, les composés volatils sont éliminés sous pression réduite et le mélange réactionnel est purifié par chromatographie sur gel de silice. L'utilisation de THF comme éluant permet de récupérer les éventuels sous-produits silylés (mélange de siloxanes et silanols). Dans un deuxième temps, l'acétate d'éthyle est utilisé comme éluant pour récupérer le composé formamide. L'acétate d'éthyle contenu dans la solution ainsi collectée est alors éliminé sous pression réduite de façon à obtenir le composé formamide analytiquement pur.

La réaction peut s'effectuer en reprenant le protocole expérimental décrit précédemment mais en utilisant uniquement l'amine réactionnelle comme solvant (pas d'ajout de solvant dans la réaction). Dans ce cas, la purification s'effectue par filtration pour éliminer le sous-produit silylé et le catalyseur, insolubles dans le mélange réactionnel à la température ambiante. Le filtrat récupéré contient le composé formamide pur. Les réactions testées jusqu'à présent par ce protocole expérimental ont permis d'obtenir une conversion totale de l'amine en composé formamide.

Un ensemble de résultats est présenté ci-dessous, donnant des exemples de conversions d'amines en composés formamides (déterminées par RMN) en utilisant dans un premier temps le phénylsilane $PhSiH_3$ et ensuite d'autres silanes, selon les conditions testées. Les structures des amines et des (pré-)catalyseurs et des silanes testés sont représentées à chaque fois.

Le schéma réactionnel est le suivant :

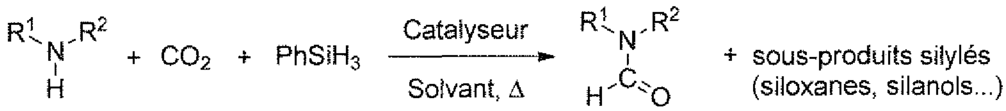


Différents (pré)catalyseurs ont été testés pour la réaction. Les résultats sont indiqués dans le tableau 1.

**Tableau 1 :**

| Amine | (Pré)Catalyseur | Equivalence Catalyseur | Solvant | Température | Temps | Conversion |
|---|---|---|---|---|---|---|
| Morpholine | TBD | 5%mol | THF | 75 °C | 24 h | 58% |
| Morpholine | MeTBD | 5%mol | THF | 75 °C | 24 h | 23% |
| Morpholine | DBU | 5%mol | THF | 75 °C | 24 h | 23% |
| Morpholine | Carbène **A** | 5%mol | THF | 75 °C | 24 h | 100% |
| Morpholine | Carbène **B** | 5%mol | benzène | 20 °C | 0,25 h | 69% |
| Morpholine | Carbène **C** | 5%mol | benzène | 20 °C | 0,25 h | 24% |
| Morpholine | Carbène **D** | 5%mol | benzène | 20 °C | 0,25 h | 25% |
| Morpholine | Carbène **E** | 5%mol | benzène | 20 °C | 0,25 h | 35% |
| Morpholine | Carbène **F** | 5%mol | benzène | 20 °C | 0,25 h | 30% |
| Morpholine | $[Fe(BH_4)_2, (H_2O)_6]$ + **$PP_3$***| 5%mol | benzène | 100 °C | 24 h | 70% |
| Morpholine | Diéthylzinc $Zn(Et)_2$ | 10%mol | benzène | 100 °C | 48 h | 87% |

* Le $PP_3$ réagit en présence du sel métallique $[Fe(BH_4)_2,(H_2O)_6]$ pour former le complexe métallique *in situ*, ledit complexe agissant comme catalyseur.

Les résultats montrent que, dans les conditions opératoires indiquées dans le tableau I, les (pré-)catalyseurs les plus actifs sont la TBD, le carbène A, le carbène B, et les complexes métalliques. Pour les autres (pré-)catalyseurs, une optimisation des conditions opératoires peut être envisagée.

Différents solvants ont aussi été testés. Les résultats, dans les conditions opératoires décrites, sont indiqués dans le tableau 2.

**Tableau 2 :**

| Amine | Catalyseur | Equivalence Catalyseur | Solvant | Température | Temps | Conversion |
|---|---|---|---|---|---|---|
| Morpholine | TBD | 5%mol | THF | 100 °C | 24 h | 65% |
| Morpholine | TBD | 5%mol | benzène | 100 °C | 24 h | 70% |
| Morpholine | TBD | 5%mol | DMSO | 100 °C | 24 h | 74% |
| Morpholine | TBD | 5%mol | $CH_3CN$ | 100 °C | 24 h | 93% |
| Morpholine | TBD | 5%mol | Sans solvant | 100 °C | 24 h | 100% |

La réaction a été testée sur des amines ayant des structures électroniques et des encombrements différents. Les résultats sont indiqués dans le tableau 3.

**Tableau 3 :**

| Amine | Catalyseur | Equivalence Catalyseur | Solvant | Température | Temps | Conversion |
|---|---|---|---|---|---|---|
| Morpholine | TBD | 5%mol | benzène | 100 °C | 24 h | 70% |
| Diisopropylamine | TBD | 5%mol | benzène | 100 °C | 24 h | 43% |
| Pipéridine | TBD | 5%mol | benzène | 100 °C | 24 h | 42% |
| Diéthylamine | TBD | 5%mol | benzène | 100 °C | 24 h | 48% |
| p-butylaniline | TBD | 5 mol % | benzène | 100°C | 24 h | 50% |
| morpholine | Carbène **A** | 5 mol % | THF | 20°C | 0,25 h | 100% |
| pipéridine | Carbène **A** | 5 mol % | THF | 20°C | 0,5 h | 100% |
| diéthylamine | Carbène **A** | 5 mol % | THF | 20°C | 1,5 h | 100% |
| imidazole | Carbène **A** | 5 mol % | THF | 20°C | 3 h | 80% |
| benzylamine | Carbène **A** | 5 mol % | THF | 20°C | 6 h | 60% |
| tertbutylamine | Carbène **A** | 5 mol % | THF | 20°C | 2 h | 100% |
| n-heptylamine | Carbène **A** | 5 mol % | THF | 20°C | 22 h | 100% |
| p-butylaniline | Carbène **A** | 5 mol % | THF | 20°C | 16 h | 100% |
| 2,6-diisopropylaniline | Carbène **A** | 5 mol % | THF | 100°C | 12 h | 95% |
| diméthylamine | Carbène **A** | 5 mol % | THF | 20°C | 24 h | 99% |
| aniline | Carbène **A** | 5 mol % | THF | 20°C | 24 h | 35% |
| diphenylméthani mine | Carbène **A** | 5 mol % | THF | 20°C | 24 h | 60% |
| 1,1-diphénylhydrazi ne | Carbène **A** | 5 mol % | THF | 20°C | 24 h | 66% |
| diphénylméthan one hydrazone | Carbène **A** | 5 mol % | THF | 20°C | 24 h | 40% |
| 1H-imidazole | Carbène **A** | 5 mol % | THF | 20°C | 24 h | 99% |
| 1,4,5,6-tetrahydropyrim idine | Carbène **A** | 5 mol % | THF | 20°C | 24 h | 99% |
| 1H-1,2,4-triazole | Carbène **A** | 5 mol % | THF | 20°C | 24 h | 10% |
| 3,5-dimethyl-1H-pyrazole | Carbène **A** | 5 mol % | THF | 20°C | 24 h | 48% |

Pour certaines amines, une optimisation des conditions opératoires peut être envisagée.

La réaction a été ensuite testée sur des silanes ayant des structures électroniques et des encombrements différents. Les différentes réactions ont été réalisées dans du benzène comme solvant et à une température de 20°C. Une optimisation des conditions opératoires peut être envisagée pour certaines réactions.

**Tableau 4 :**

| Amine | Catalyseur | Equivalence Catalyseur | Silane | Equivalence Silane | Temps | Conversion |
|---|---|---|---|---|---|---|
| Morpholine | Carbène **A** | 5%mol | $(EtO)_3SiH$ | 3 éq. | 24 h | 28% |
| Morpholine | Carbène **A** | 5%mol | TMDS | 1,5 éq. | 24 h | 43% |

(suite)

| Amine | Catalyseur | Equivalence Catalyseur | Silane | Equivalence Silane | Temps | Conversion |
|---|---|---|---|---|---|---|
| Morpholine | Carbène **A** | 5%mol | PMHS | 3 éq. | 24 h | 90% |
| Diméthylamine | Carbène **A** | 5%mol | PMHS | 3 éq. | 24 h | 90% |
| n-héptylamine | Carbène **A** | 5 mol % | PMHS | 3 éq. | 24 h | 67% |
| benzylamine | Carbène **A** | 5 mol % | PMHS | 9 éq. | 24 h | 70% |
| aniline | Carbène **A** | 5 mol % | PMHS | 3 éq. | 24 h | 83% |
| Diphénylméthan imine | Carbène **A** | 5 mol % | PMHS | 3 éq. | 24 h | 35% |
| 1,1-diphénylhydrazi ne | Carbène **A** | 5 mol % | PMHS | 3 éq. | 24 h | 83% |
| 3,5-diméthyl-1H-pyrazole | Carbène **A** | 5 mol % | PMHS | 3 éq. | 24 h | 29% |

Le nombre d'équivalents molaires de silane indiqué est par rapport à l'amine.
Les abréviations utilisées dans les tableaux sont :

Morpholine
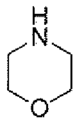
TBD
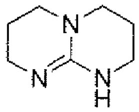
MeTBD
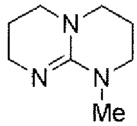
DBU
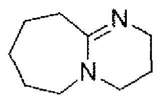
Carbène **A**
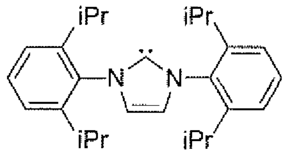

Morpholine
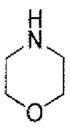
Carbène **A**
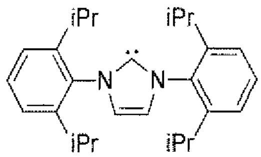
Carbène **B**
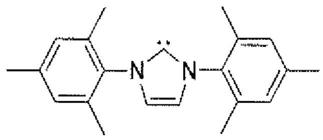
Carbène **C**
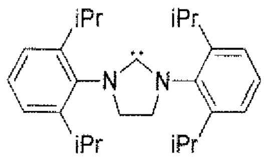

Carbène **D**
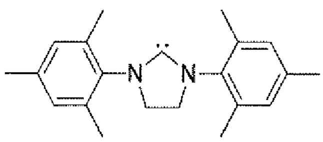
Carbène **E**
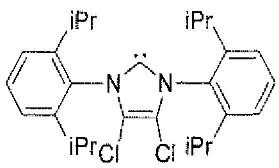
Carbène **F**
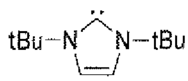

diphenylmethanimine
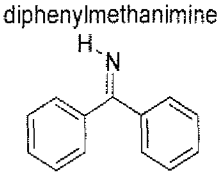
1,1-diphenylhydrazine
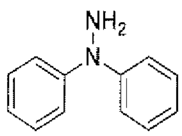
diphenylmethanone hydrazone
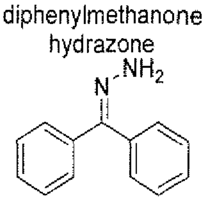
1,4,5,6-tetrahydropyrimidine
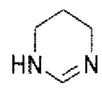

1H-imidazole
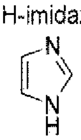
3,5-dimethyl-1H-pyrazole
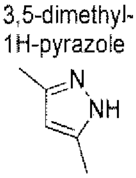
1H-1,2,4-triazole
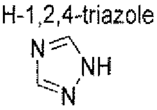
PMHS (polyméthylhydrosiloxane)
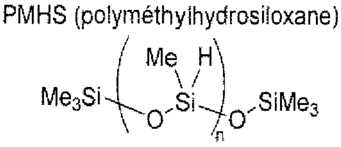

1,1,3,3-tetramethyldisiloxane

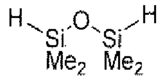

PP3

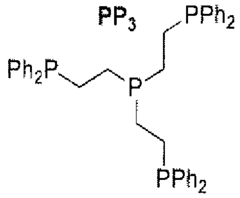

Ces résultats montrent que la préparation des composés formamides par le procédé de l'invention est suffisamment flexible pour convertir efficacement et avec une excellente sélectivité une grande variété d'amines : les amines primaires et secondaires, aliphatiques, aromatiques et hétérocycliques, en composés formamides, et ce dans des conditions de pressions de $CO_2$ et de températures de réaction douces.

Plus particulièrement, en l'absence de solvant, la préparation des composés formamides selon le procédé de l'invention se fait avec un excellent rendement et une excellente sélectivité.

**EXEMPLE 2 : Synthèse d'un inhibiteur marqué $^{14}C$ de la phosphodiesterase-4 (PDE-4)**

Synthèse du composé formamide marqué $^{14}C$

Le composé formamide de formule $Bu_2N^{14}CHO$ a été synthétisé selon le protocole expérimental indiqué dans l'exemple 1, en utilisant du $CO_2$ marqué obtenu par le procédé décrit par R. Voges, J. R. Heys, T. Moenius, "Preparation of Compounds Labeled with Tritium and Carbon-14". Wiley-VCH: Chippenham (UK), 2009, et dans les mêmes conditions de catalyseur, de solvant, de température et de temps que celles indiquées pour le diisopropylamine (Tableau 3).

Synthèse d'un inhibiteur marqué $^{14}C$ de la phosphodiesterase-4 (PDE-4)

Le composé formamide de formule $Bu_2N^{14}CHO$ a ensuite été utilisé dans la synthèse de phosphodiesterase-4 (PDE-4) selon la synthèse décrite par J. Z. Ho and coll., Helvetica Chimica Acta, 2005, 88, p.1040.

**EXEMPLE 3 : Synthèse de l'irbesartan marqué $^{14}C$.**

Synthèse du diméthylformamide marqué $^{14}C$

Le diméthylformamide marqué de formule $(CH_3)_2N^{14}CHO$ a été synthétisé selon le protocole expérimental indiqué dans l'exemple 1, en utilisant du $CO_2$ marqué obtenu par le procédé décrit par R. Voges, J. R. Heys, T. Moenius, "Preparation of Compounds Labeled with Tritium and Carbon-14". Wiley-VCH: Chippenham (UK), 2009, et dans les mêmes conditions de catalyseur (carbène A), de solvant, de température et de temps que celles indiquées pour le diéthylamine (Tableau 3).

Synthèse de l'irbesartan marqué $^{14}C$ (médicament anti-hypertenseur)

Le diméthylformamide marqué de formule $(CH_3)_2N^{14}CHO$ a ensuite été utilisé dans la synthèse de l'irbésartan selon la synthèse décrite par I. V. Ekhato, S. Bonacorsi Jr., J., Label Compd. Radiopharm, 2011, 54, p.202-205.

**EXEMPLE 4 : Synthèse de la 5-azacytidine marquée $^{14}C$**

Synthèse du diméthylformamide marqué $^{14}C$

La diméthylformamide marqué de formule $(CH_3)_2N^{14}CHO$ a été synthétisé selon le protocole de l'exemple 2.

Synthèse de la 5-azacytidine marquée $^{14}C$ (agent antitumoral et antileucémique)

La dimethylformamide marqué de formule $(CH_3)_2N^{14}CHO$ a ensuite été utilisé dans la synthèse de la 5-azacytidine selon la synthèse décrite par Kenneth K. Chan, James A. Staroscik, Journal of Medicinal Chemistry, 1977, Vol. 20, No. 4, p.598.

## Revendications

**1.** Procédé de préparation de composés formamides de formule (I) :

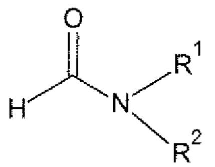

(I)

dans laquelle

■ $R^1$ et $R^2$, représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, une aldimine de formule $-N=CHR^6$, une cétimine de formule $-N=CR^6R^7$, lesdits groupes alkyle, alcényle, alcynyle, aryle, hétéroaryle, hétérocycle, silylé, siloxy et amino étant éventuellement substitués, ou
■ $R^1$ et $R^2$, pris ensemble avec l'atome d'azote auquel ils sont liés forment un hétérocycle éventuellement substitué, ou
■ $R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$,
et
■ $R^6$ et $R^7$, représentant, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle, alcynyle, aryle, hétéroaryle, hétérocycle, silylé, siloxy et amino étant éventuellement substitués,

**caractérisé en ce que** l'on fait réagir une amine de formule (II) dans laquelle $R^1$ et $R^2$ sont tels que définis ci-dessus,

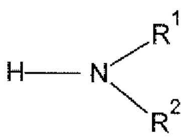

(II)

avec du $CO_2$, en présence d'un catalyseur et d'un composé silane de formule (III)

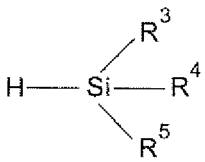

(III)

dans laquelle

■ $R^3$, $R^4$ et $R^5$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
■ $R^5$ est tel que défini ci-dessus et $R^3$ et $R^4$, pris ensemble avec l'atome de silicium auquel ils sont liés forment un hétérocycle silylé éventuellement substitué.

**2.** Procédé selon la revendication 1, **caractérisé en ce que** dans l'amine de formule (II), $R^1$ et $R^2$, représentent, indépendamment l'un de l'autre, un atome d'hydrogène, un groupe alkyle, un groupe aryle, un groupe hétéroaryle, un groupe amino, lesdits groupes alkyle, amino, aryle et hétéroaryle étant éventuellement substitués, ou
$R^1$ et $R^2$, pris ensemble avec l'atome d'azote auquel ils sont liés forment un hétérocycle éventuellement substitué ; ou

$R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$, dans laquelle $R^6$ et $R^7$, représentent, indépendamment l'un de l'autre, un atome d'hydrogène, un groupe alkyle, un groupe aryle, un groupe hétéroaryle, un hétérocycle, lesdits groupes alkyle, aryle, hétéroaryle et hétérocycleétant éventuellement substitués.

**3.** Procédé selon l'une des revendications 1 ou 2, **caractérisé en ce que** dans le composé silane de formule (III), $R^3$, $R^4$ et $R^5$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

**4.** Procédé selon l'une quelconque des revendications 1 à 3, **caractérisé en ce que** le catalyseur est choisi parmi les catalyseurs organiques ou les catalyseurs métalliques choisi parmi les sels ou les complexes métalliques.

**5.** Procédé selon la revendication 4, **caractérisé en ce que** le catalyseur organique est :

- une amine secondaire ou tertiaire choisie parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou
- un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant sous la forme de sels de chlorure.

**6.** Procédé selon la revendication 4, **caractérisé en ce que** le catalyseur métallique est choisi parmi les sels ou complexes de métaux de transition choisis parmi le nickel, le fer, le cobalt, le zinc, le cuivre, le rhodium, le ruthénium, le platine, le palladium, l'iridium.

**7.** Procédé selon la revendication 6, **caractérisé en ce que** le catalyseur métallique est choisi parmi $Fe(BH_4)_2.6H_2O$, CuCl, $ZnEt_2$.

**8.** Procédé selon l'une quelconque des revendications 1 à 7, **caractérisé en ce que** la réaction est effectuée sous une pression de $CO_2$, comprise entre 1 et 50 bars, bornes incluses.

**9.** Procédé selon l'une quelconque des revendications 1 à 8, **caractérisé en ce que** la réaction est effectuée à une température comprise entre 25 et 150°C, bornes incluses.

**10.** Procédé selon l'une quelconque des revendications 1 à 9, **caractérisé en ce que** la réaction est effectuée pendant une durée de 5 minutes à 72 heures, bornes incluses.

**11.** Procédé selon l'une quelconque des revendications 1 à 10, **caractérisé en ce que** la réaction est effectuée dans un ou un mélange d'au moins deux solvant(s) choisi(s) parmi :

- les éthers choisis parmi l'éther diéthylique, ou le tetrahydrofuran (THF) ;
- les hydrocarbures choisis parmi le benzène, ou le toluène ;
- les solvants azotés choisis parmi la pyridine, ou l'acétonitrile ;
- les sulfoxydes, choisis parmi le diméthylesulfoxyde ;
- les halogénures d'alkyle choisis parmi le chloroforme, ou le chlorure de méthylène.

**12.** Procédé selon l'une quelconque des revendications 1 à 10, **caractérisé en ce que** le solvant est l'amine de formule (II).

**13.** Procédé selon l'une quelconque des revendications 1 à 12, **caractérisé en ce que** le rapport molaire entre le composé silane de formule (III) et l'amine de formule (II) est de 0,5 à 5, bornes incluses.

**14.** Procédé selon l'une quelconque des revendications 1 à 13, **caractérisé en ce que** la quantité de catalyseur est de

0,001 à 1 équivalent molaire, bornes incluses, par rapport à l'amine de formule (II).

**15.** Utilisation d'un procédé de préparation de composés formamides de formule (I) selon l'une quelconque des revendications 1 à 14, dans la fabrication de vitamines, de produits pharmaceutiques, de colles, de fibres acryliques, de cuirs synthétiques, de pesticides, et d'engrais.

**16.** Procédé de préparation de composés formamides marqués de formule (I') :

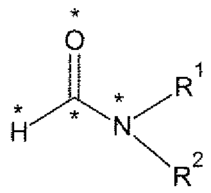

(I')

dans laquelle

- ■ $R_1$ et $R_2$ sont tels que définis à l'une des revendications 1 ou 2,
- ■ $H^*$ représente un atome d'hydrogène ($^1H$), le deutérium ($^2H$) ou le tritium ($^3H$),
- ■ $O^*$ représente un atome d'oxygène ($^{16}O$), un isotope $^{17}O$ ou $^{18}O$,
- ■ $C^*$ représente un atome de carbone ($^{12}C$), un isotope $^{11}C$, $^{13}C$ ou $^{14}C$,
- ■ $N^*$ représente un atome d'azote ($^{14}N$), un isotope $^{15}N$,

**caractérisé en ce que** l'on fait réagir une amine de formule (II') dans laquelle $R^1$ et $R^2$ sont tels que définis à l'une des revendications 1 ou 2 et $N^*$ est tel que défini ci-dessus,

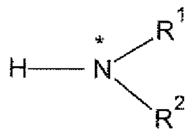

(II')

avec du $C^*O_2^*$ dans lequel $C^*$ et $O^*$ sont tels que définis ci-dessus, en présence d'un catalyseur tel que défini à l'une des revendications 4 à 7 et d'un composé silane de formule (III')

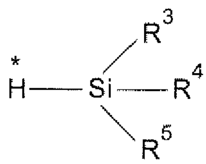

(III')

dans laquelle

- ■ $R^3$, $R^4$ et $R^5$ sont tels que définis à l'une des revendications 1 ou 3 et $H^*$ est tel que défini ci-dessus.

**Patentansprüche**

**1.** Verfahren zur Herstellung von Formamidverbindungen der Formel (I):

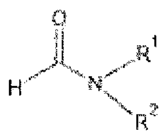

(I)

wobei

- $R^1$ und $R^2$ unabhängig voneinander ein Wasserstoffatom, eine Alkylgruppe, eine Alkenylgruppe, eine Alkenylgruppe, eine Arylgruppe, eine Heteroarylgruppe, einen Heterozyklus, eine Silylgruppe, eine Siloxygruppe, eine Aminogruppe, ein Aldimin der Formel -N=CHR$^6$, ein Ketimin der Formel -N=CR$^6$R$^7$ darstellen, wobei die Alkyl-, Alkenyl-, Alkynyl-, Aryl-, Heteroaryl-, heterozyklischen, Silyl-, Siloxy- und Aminogruppen eventuell substituiert sind, oder
- $R^1$ und $R^2$, gemeinsam herangezogen mit dem Stickstoffatom, mit dem sie verbunden sind, einen eventuell substituierten Heterozyklus bilden, oder
- $R^1$ und $R^2$ mit dem Stickstoffatom, mit dem sie verbunden sind, eine Kohlenstoff-Stickstoff-Doppelbindung (N=C) bilden, um zu einem Aldimin der Formel -N=CHR$^6$ oder zu einem Ketimin der Formel -N=CR$^6$R$^7$ zu führen, und
- $R^6$ und $R^7$ unabhängig voneinander ein Wasserstoffatom, eine Alkylgruppe, eine Alkenylgruppe, eine Alkynylgruppe, eine Aroylgruppe, eine Heteroarylgruppe, einen Heterozyklus, eine Silylgruppe, eine Siloxygruppe, eine Aminogruppe darstellen, wobei die Alkyl-, Alkenyl-, Alkynyl-, Aryl-, Heteroaryl-, heterozyklischen, Silyl-, Siloxy- und Aminogruppen eventuell substituiert sind,

**dadurch gekennzeichnet, dass** man ein Amin der Formel (II) in Reaktion versetzt, wobei $R^1$ und $R^2$ wie oben definiert sind,

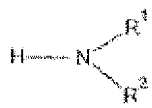
(II)

mit $CO_2$, in Anwesenheit eines Katalysators und einer Silanverbindung der Formen (III),

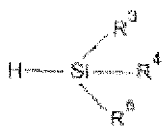
(III)

wobei

- $R^3$, $R^4$ und $R^5$ unabhängig voneinander ein Wasserstoffatom, ein Halogenatom, eine Hydroxylgruppe, eine Alkylgruppe, eine Alkenylgruppe, eine Alkynylgruppe, eine Alkoxygruppe, eine Silylgruppe, eine Siloxygruppe, eine Arylgruppe, eine Aminogruppe darstellen, wobei die Alkyl-, Alkenyl-, Alkynyl-, Alkoxy-, Silyl-, Siloxy-, Aryl- und Aminogruppen eventuell substituiert sind, oder
- $R^5$ derart wie oben definiert ist und $R^3$ und $R^4$, gemeinsam herangezogen mit dem Siliziumatom, mit dem sie verbunden sind, einen eventuell substituierten Silyl-Heterozyklus bilden.

2. Verfahren nach Anspruch 1, **dadurch gekennzeichnet, dass** im Amin der Formel (II) $R^1$ und $R^2$ unabhängig voneinander ein Wasserstoffatom, eine Alkylgruppe, eine Arylgruppe, eine Heteroarylgruppe, eine Aminogruppe darstellen, wobei die Alkyl-, Amino-, Aryl- und Heteroarylgruppen eventuell substituiert sind, oder
$R^1$ und $R^2$, gemeinsam herangezogen mit dem Stickstoffatom, mit dem sie verbunden sind, einen eventuell substituierten Heterozyklus bilden, oder
$R^1$ und $R^2$ mit dem Stickstoffatom, mit dem sie verbunden sind, eine Kohlenstoff-Stickstoff-Doppelbindung (N=C) bilden, um zu einem Aldimin der Formel -N=CHR$^6$ oder zu einem Ketimin der Formel -N=CR$^6$R$^7$ zu führen, wobei $R^6$ und $R^7$ unabhängig voneinander ein Wasserstoffatom, eine Alkylgruppe, eine Arylgruppe, eine Heteroarylgruppe, einen Heterozyklus darstellen, wobei die Alkyl-, Aryl-, Heteroaryl- und heterozyklischen Gruppen eventuell substituiert sind.

3. Verfahren nach einem der Ansprüche 1 oder 2, **dadurch gekennzeichnet, dass** in der Silanverbindung der Formel (III) $R^3$, $R^4$ und $R^5$ unabhängig voneinander ein Wasserstoffatom, eine Alkylgruppe, eine Alkoxygruppe, eine Arylgruppe, eine Silylgruppe, eine Siloxygruppe darstellen, wobei die Alkyl-, Alkoxy-, Silyl-, Siloxy- und Arylgruppen eventuell substituiert sind.

4. Verfahren nach einem der Ansprüche 1 bis 3, **dadurch gekennzeichnet, dass** der Katalysator aus den organischen Katalysatoren oder den Metallkatalysatoren, die aus den Salzen oder den Metallkomplexen ausgewählt sind, ausgewählt ist.

5. Verfahren nach Anspruch 4, **dadurch gekennzeichnet, dass** der organische Katalysator ist:

   - ein sekundäres oder tertiäres Amin, ausgewählt aus dem Triazabicyclodecen (TBD), dem N-methyltriazabicyclodecen (MeTBD), dem 1,8-Diazabicyclo[5.4.0]undec-7-en (DBU), dem Trimethylamin, dem Triethylmain, dem Piperidin, dem 4-Dimethylaminopyridin (DMAP), dem 1,4-Diazabicyclo[2.2.2]octan (DABCO), dem Prolin, dem Phenylalanin, einem Thiazoliumsalz, dem *N*-Diisopropylethylamin (DIPEA oder DIEA), oder
   - ein Carben aus einem Imidazoliumsalz, ausgewählt aus den Salzen von 1,3-bis(2,6-diisopropylphenyl)-1H-imidazol-3-ium (Carben A), 1,3-bis(2,6-diisopropylphenyl)-4,5-dihydro-1H-imidazol-3-ium (Carben C), 1,3-bis(2,4,6-trimethylphenyl)-1H-imidazol-3-ium (Carben B), 1,3-bis(2,4,6-trimethylphenyl)-4,5-dihydro-1H-imidazol-3-ium (Carben D), 4,5-dichlor-1,3-bis(2,6-diisopropylphenyl)-1H-imidazol-3-ium (Carben E), 1,3-di-tert-butyl-1H-imidazol-3-ium (Carben F), 1,3-di-tert-butyl-4,5-dihydro-1H-imidazol-3-ium, wobei die Salze in Form von Chloridsalzen sind.

6. Verfahren nach Anspruch 4, **dadurch gekennzeichnet, dass** der Metallkatalysator aus den Salzen oder Komplexen von Übergangsmetallen ausgewählt ist, die aus dem Nickel, dem Eisen, dem Kobalt, dem Zink, dem Kupfer, dem Rhodium, dem Ruthenium, dem Platin, dem Palladium, dem Iridium ausgewählt sind.

7. Verfahren nach Anspruch 6, **dadurch gekennzeichnet, dass** der Metallkatalysator aus $Fe(BH_4)_2.6H_20$, CuCl, $ZnEt_2$ ausgewählt ist.

8. Verfahren nach einem der Ansprüche 1 bis 7, **dadurch gekennzeichnet, dass** die Reaktion unter Druck von $CO_2$ zwischen 1 und 50 bar inklusive, Grenzen inbegriffen, durchgeführt wird.

9. Verfahren nach einem der Ansprüche 1 bis 8, **dadurch gekennzeichnet, dass** die Reaktion bei einer Temperatur zwischen 25 und 150 °C inklusive, Grenzen inbegriffen, durchgeführt wird.

10. Verfahren nach einem der Ansprüche 1 bis 9, **dadurch gekennzeichnet, dass** die Reaktion während einer Dauer von 5 Minuten bis 72 Stunden, Grenzen inbegriffen, durchgeführt wird.

11. Verfahren nach einem der Ansprüche 1 bis 10, **dadurch gekennzeichnet, dass** die Reaktion in einem Lösungsmittel oder in einem Gemisch von mindestens zwei Lösungsmitteln durchgeführt wird, das/die ausgewählt ist/sind aus:

    - den Ethern, ausgewählt aus dem Diethylenether oder dem Tetrahyrofutan (THF),
    - den Kohlenwasserstoffen, ausgewählt aus dem Benzol, oder dem Toluen,
    - den stickstoffhaltigen Lösungsmitteln, ausgewählt aus dem Pyridin, oder dem Aceton itril,
    - den Sulfoxiden, ausgewählt aus dem Dimethylsulfoxid,
    - den Alkylhalogeniden, ausgewählt aus dem Chloroform, oder dem Methylenchlorid.

12. Verfahren nach einem der Ansprüche 1 bis 10, **dadurch gekennzeichnet, dass** das Lösungsmittel das Amin der Formel (II) ist.

13. Verfahren nach einem der Ansprüche 1 bis 12, **dadurch gekennzeichnet, dass** das molare Verhältnis zwischen der Silanverbindung der Formel (III) und dem Amin der Formel (II) von 0,5 bis 5, Grenzen inklusive, beträgt.

14. Verfahren nach einem der Ansprüche 1 bis 13, **dadurch gekennzeichnet, dass** die Katalysatormenge von 0,001 bis 1 molares Äquivalent, Grenzen inklusive, im Verhältnis zum Amin der Formel (II) beträgt.

15. Verwendung eines Verfahrens zur Herstellung von Formamidverbindungen der Formel (I) nach einem der Ansprüche 1 bis 14 bei der Herstellung von Vitaminen, pharmazeutischen Produkten, Klebern, Acrylfasern, Kunstledern, Pestiziden und Dünger.

16. Verfahren zur Herstellung von Formamidverbindungen der Formel (I'):

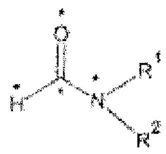

(I')

wobei

- $R_1$ und $R_2$ wie in einem der Ansprüche 1 oder 2 definiert sind,
- H* ein Wasserstoffatom ($^1H$), das Deuterium ($^2H$) oder das Tritium ($^3H$) darstellt,
- O* ein Sauerstoffatom ($^{16}O$), ein Isotop $^{17}O$ oder $^{18}O$ darstellt,
- C* ein Kohlenstoffatom ($^{12}C$), ein Isotop $^{11}C$, $^{13}C$ oder $^{14}C$ darstellt,
- N* ein Stickstoffatom ($^{14}N$), ein Isotop $^{15}N$ darstellt,

**dadurch gekennzeichnet, dass** ein Amin der Formel (II') in Reaktion versetzt wird, wobei $R^1$ und $R^2$ wie in einem der Ansprüche 1 oder 2 definiert sind und N* wie hier oben definiert ist,

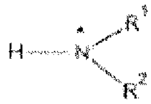

(II')

mit $C^*O_2^*$, wobei C* und O* wie hier oben definiert sind, in Anwesenheit eines Katalysators wie in einem der Ansprüche 4 bis 7 definiert und einer Silanverbindung der Formel (III')

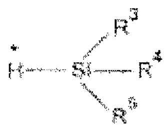

(III')

wobei

- $R^3$, $R^4$ und $R^5$ wie in einem der Ansprüche 1 oder 3 definiert sind und H* wie hier oben definiert ist.

## Claims

**1.** A process for preparing formamide compounds of formula (I)

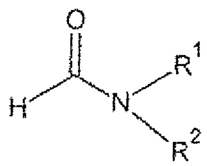

(I)

wherein:

- $R^1$ and $R^2$ are each independently a hydrogen atom, an alkyl group, an alkenyl group, an alkynyl group, an aryl group, a heteroaryl group, a heterocycle, a silylated group, a siloxy group, an amino group, an aldimine of formula $-N=CHR^6$, a ketimine of formula $-N=CR^6R^7$, said alkyl, alkenyl, alkynyl, aryl, heteroaryl, heterocycle, silylated, siloxy and amino groups optionally being substituted; or
- $R^1$ and $R^2$ together with the nitrogen atom to which they are attached form an optionally substituted heterocycle; or
- $R^1$ and $R^2$ together with the nitrogen atom to which they are attached form a double carbon-nitrogen bond (N=C) leading to an aldimine of formula $-N=CHR^6$ or to a ketimine of formula $-N=CR^6R^7$;

and

- $R^6$ and $R^7$ are each independently a hydrogen atom, an alkyl group, a alkenyl group, an alkynyl group, an aryl group, a heteroaryl group, a heterocycle, a silylated group, a siloxy group, an amino group, said alkyl, alkenyl, alkynyl, aryl, heteroaryl, heterocycle, silylated, siloxy and amino groups optionally being substituted,

**characterized in that** an amine of formula (II) in which $R^1$ and $R^2$ are such as defined above

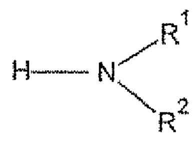

(II)

is reacted with $CO_2$ in the presence of catalyst and a silane compound of formula (III)

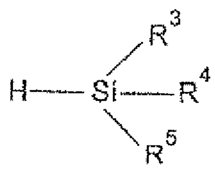

(III)

wherein:

- $R^3$, $R^4$ and $R^5$ are each independently a hydrogen atom, a halogen atom, a hydroxyl group, an alkyl group, an alkenyl group, an alkynyl group, an alkoxy group, a silylated group, a siloxy group, an aryl group, an amino group, said alkyl, alkenyl, alkynyl, alkoxy, silylated, siloxy, aryl and amino groups optionally being substituted; or
- $R^5$ is such as defined above and $R^3$ and $R^4$ together with the silicon atom to which they are attached form an optionally substituted silylated heterocycle.

**2.** The process according to claim 1 **characterized in that** in the amine of formula (II), $R^1$ and $R^2$ are each independently a hydrogen atom, an alkyl group, an aryl group, a heteroaryl group, an amino group, said alkyl, amino, aryl and heteroaryl groups optionally being substituted; or
$R^1$ and $R^2$ together with the nitrogen atom to which they are attached form an optionally substituted heterocycle; or
$R^1$ and $R^2$ together with the nitrogen atom to which they are attached form a double carbon-nitrogen bond (N=C) leading to an aldimine of formula $-N=CHR^6$ or to a ketimine of formula $-N=CR^6R^7$ wherein $R^6$ and $R^7$ are each independently a hydrogen atom, an alkyl group, an aryl group, a heteroaryl group, a heterocycle, said alkyl, aryl, heteroaryl and heterocycle groups optionally being substituted.

**3.** The process according to one of claims 1 or 2 **characterized in that** in the silane compound of formula (III), $R^3$, $R^4$ and $R^5$ are each independently a hydrogen atom, alkyl group, alkoxy group, aryl group, silylated group, siloxy group, said alkyl, alkoxy, silylated, siloxy and aryl groups optionally being substituted.

**4.** The process according to any of claims 1 to 3 **characterized in that** the catalyst is selected from among organic catalysts or metal catalysts selected among metal salts or complexes.

**5.** The process according to claim 4 **characterized in that** the organic catalyst is:

- a secondary or tertiary amine selected among triazabicyclodecene (TBD); N-methyltriazabicyclodecene (MeTBD), 1,8-Diazabicyclo[5.4.0]undec-7-ene (DBU), trimethylamine, trimethylamine, piperidine, 4-dimethylaminopyridine (DMAP), 1,4-diazabicyclo[2.2.2]octane (DABCO), proline, phenylalanine, thiazolium salt, *N*-diisopropylethylamine (DIPEA or DIEA); or
- a carbene derived from an imidazolium salt selected among the salts of 1,3-bis(2,5-diisopropylphenyl)-1H-imidazol-3-ium (carbene A), 1,3-bis(2,6-diisopropylphenyl)-4,5-dihydro-1H-imidazol-3-ium (carbene C), 1,3-bis(2,4,6-trimethylphenyl)-1H-imidazol-3-ium (carbene B), 1,3-bis(2,4,6-trimethylphenyl)-4,5-dihydro-1H-imidazol-3-ium (carbene D), 4,5-dichloro-1,3-bis(2,6-diisopropylphenyl)-1H-imidazol-3-ium (carbene E), 1,3-di-tert-butyl-1H-imidazol-3-ium (carbene F), 1,3-di-tert-butyl-4,5-dihydro-1H-imidazol-3-ium, said salts being in the form of chloride salts.

**6.** The process according to claim 4 **characterized in that** the metal catalyst is selected among salts or complexes of transition metals selected among nickel, iron, cobalt, zinc, copper, rhodium, ruthenium, platinum, palladium, iridium.

**7.** The process according to claim 6 **characterized in that** the metal catalyst is selected among $Fe(BH)_4)_2.6H_2O$, CuCl, $ZnEt_2$.

**8.** The process according to any of claims 1 to 7 **characterized in that** the reaction is conducted under $CO_2$ pressure of between 1 and 50 bars, limits included.

**9.** The process according to any of claims 1 to 8 **characterized in that** the reaction is conducted at a temperature of between 25 and 150°C, limits included.

**10.** The process according to any of claims 1 to 9 **characterized in that** the reaction is conducted for a time of 5 minutes to 72 hours, limits included.

**11.** The process according to any of claims 1 to 10 **characterized in that** the reaction is conducted in one or a mixture of at least two solvents selected among:

- ethers selected among diethyl ether or tetrahydrofuran (THF);
- hydrocarbons selected among benzene or toluene;
- nitrogen-containing solvents selected among pyridine or acetonitrile;
- sulfoxides selected among diemthylsulfoxide;
- alkyl halides selected among chloroform or methylene chloride.

**12.** The process according to any of claims 1 to 10 **characterized in that** the solvent is the amine of formula (II).

**13.** The process according to any of claims 1 to 12 **characterized in that** the molar ratio between the silane compound of formula (III) and the amine of formula (II) is 0.5 to 5, limits included.

**14.** The process according to any of claims 1 to 13 **characterized in that** the amount of catalyst is 0.001 to 1 molar equivalent, limits included, relative to the amine of formula (II).

**15.** The use of a process to prepare formamide compounds of formula (I) according to any of claims 1 to 14 for the manufacture of vitamins, pharmaceutical products, glues, acrylic fibres, synthetic leather, pesticides and fertilisers.

**16.** A process to prepare labelled formamide compounds of formula (I'):

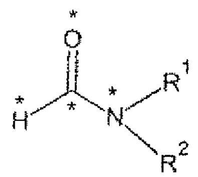

(I')

wherein:

- $R^1$ and $R^2$ are such as defined in either of claims 1 or 2;
- H* is a hydrogen atom ($^1H$), deuterium ($^2H$) or tritium ($^3H$);
- O* is an oxygen atom ($^{16}O$), an isotope $^{17}O$ or $^{18}O$;
- C* is a carbon atom ($^{12}C$), an isotope $^{11}C$, $^{13}C$ or $^{14}C$;
- N* is a nitrogen atom ($^{14}N$), an isotope $^{15}N$,

**characterized in that** an amine of formula (II') wherein $R^1$ and $R^2$ are such as defined in either of claims 1 or 2 and N* is such as defined above

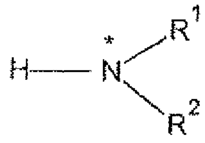

(II')

is reacted with $C^*O_2^*$ wherein C* and O* are such as defined above, in the presence of a catalyst such as defined in one of claims 4 to 7 and of a silane compound of formula (III')

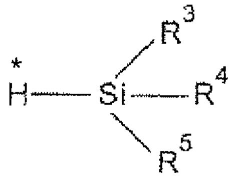

(III')

where:

- $R^3$, $R^4$ and $R^5$ are such as defined in one of claims 1 or 3 and H* is such as defined above.

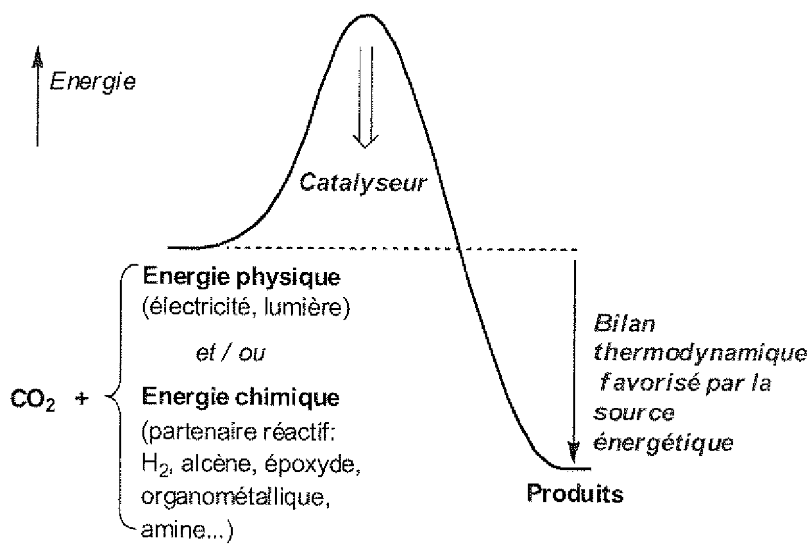
Energie
Catalyseur
Energie physique
(électricité, lumière)
et / ou
$CO_2$ +
Energie chimique
(partenaire réactif:
$H_2$, alcène, époxyde,
organométallique,
amine...)
Bilan
thermodynamique
favorisé par la
source
énergétique
Produits

Figure 1

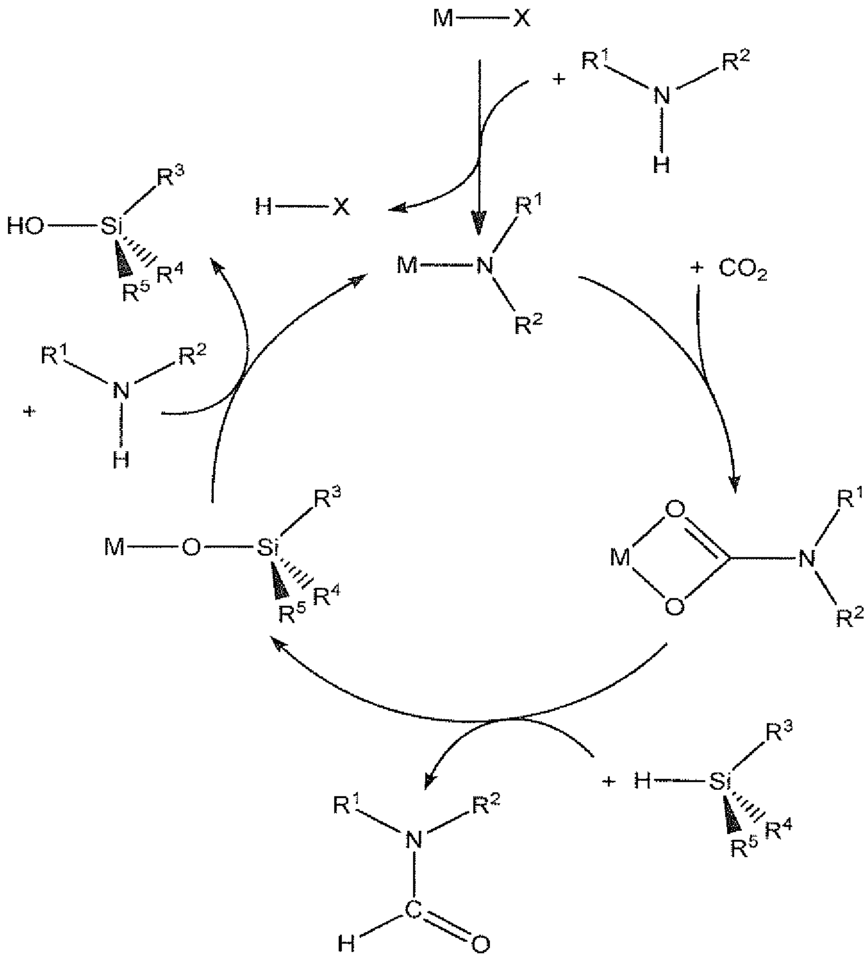


Figure 2

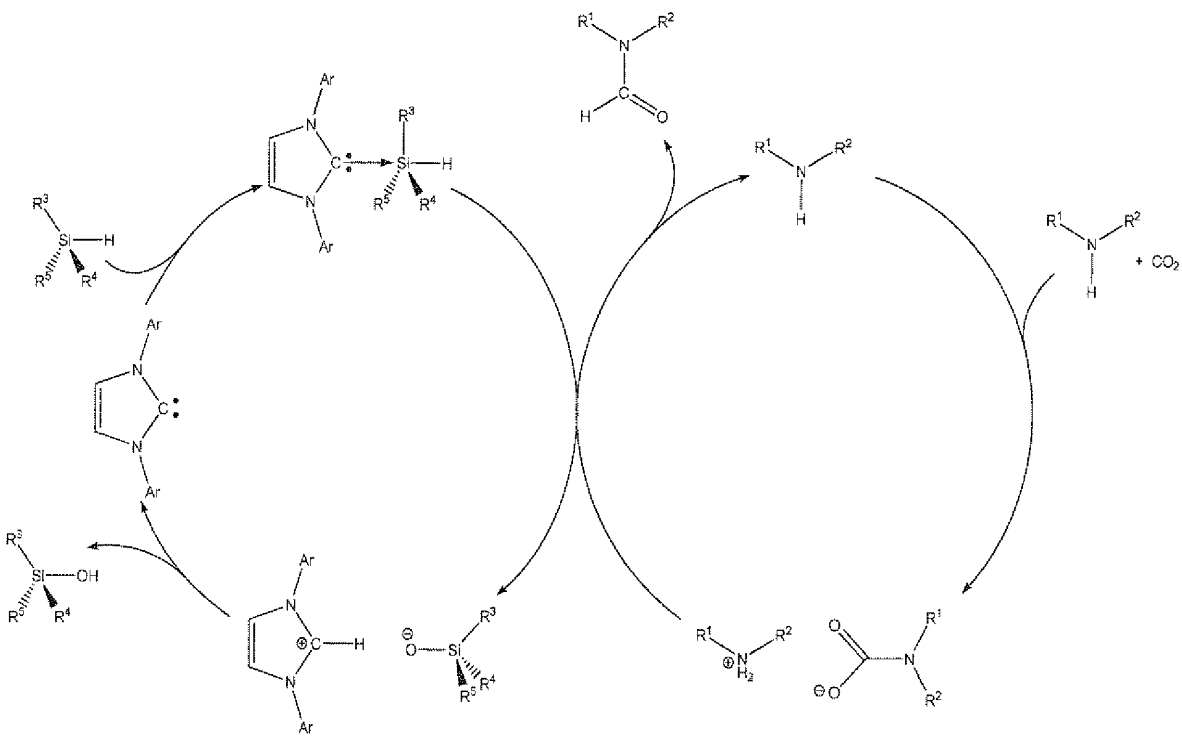

Figure 3

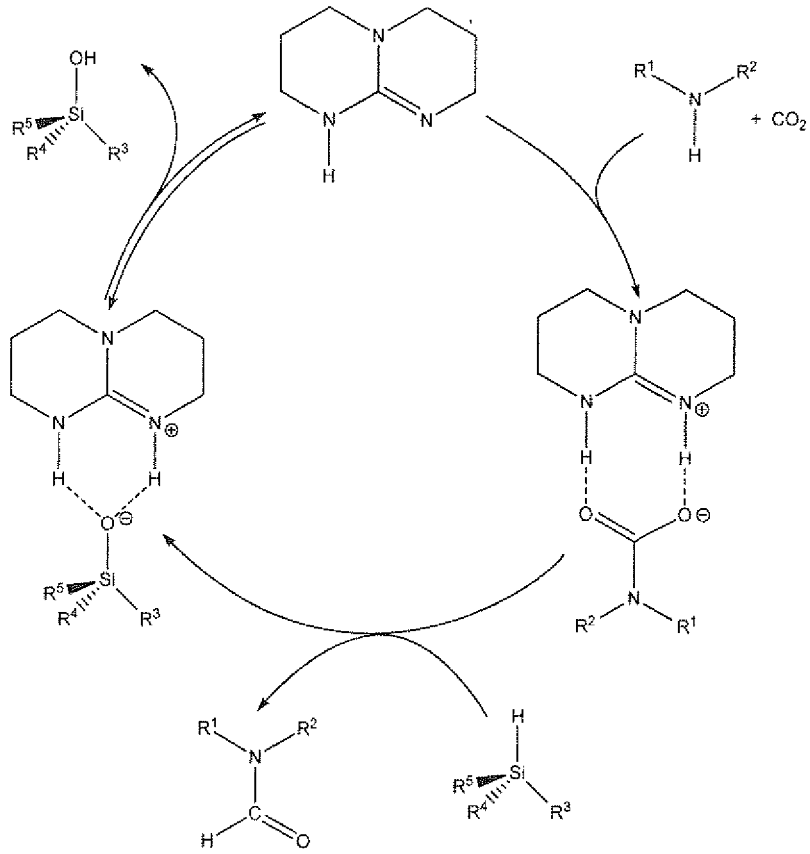

Figure 4

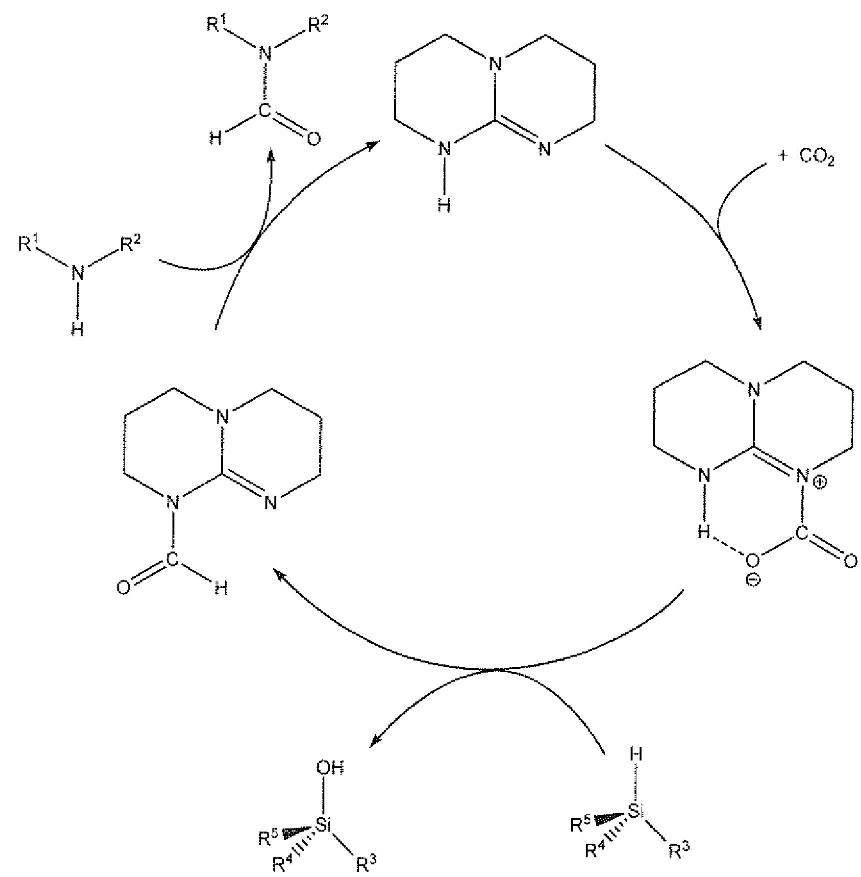


Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 52036617 B, Kiso Y.
- JP 62255456 B, Fujiwara Y.
- EP 652202 A, Ikariya, T.


### Littérature non-brevet citée dans la description


- **MORRIS, A. J. ; MEYER, G. J. ; FUJITA, E.** *Accounts Chem Res,* 2009, vol. 42, 1983
- **RIDUAN, S. N. ; ZHANG, Y. G. ; YING, J. Y.** *Angewandte Chemie-International Edition,* 2009, vol. 48, 3322
- **SAKAKURA, T. ; CHOI, J. C. ; YASUDA, H.** *Chem Rev,* 2007, vol. 107, 2365
- **GREENBERG, A. B. ; C. M., LIEBMAN, J. F.** The Amide Linkage: Structural Significance in Chemistry, Biochemistry, and Materials Science. Wiley-Interscience, 2002
- **SCHREINER, S. ; YU, J. Y. ; VASKA, L.** *Inorganica Chimica Acta,* 1988, vol. 147, 139
- **SCHREINER, S. ; YU, J. Y. ; VASKA, L.** *Journal of the Chemical Society-Chemical Communications,* 1988, 602
- **VASKA, L. ; SCHREINER, S. ; FELTY, R. A. ; YU, J. Y.** *Journal of Molecular Catalysis,* 1989, vol. 52, L11
- **YU, J. Y. ; SCHREINER, S. ; VASKA, L.** *Inorganica Chimica Acta,* 1990, vol. 170, 145
- **JESSOP, P. G. ; HSIAO, Y. ; IKARIYA, T. ; NOYORI, R.** *Journal of the American Chemical Society,* 1994, vol. 116, 8851
- **JESSOP, P. G. ; HSIAO, Y. ; IKARIYA, T. ; NOYORI, R.** *Journal of the American Chemical Society,* 1996, vol. 118, 344
- **MUNSHI, P. ; HELDEBRANT, D. J. ; MCKOON, E. P. ; KELLY, P. A. ; TAI, C. C. ; JESSOP, P. G.** *Tetrahedron Letters,* 2003, vol. 44, 2725
- **SCHMID, L. ; ROHR, M. ; BAIKER, A.** *Chemical Communications,* 1999, 2303
- **FEDERSEL, C. ; BODDIEN, A. ; JACKSTELL, R. ; JENNERJAHN, R. ; DYSON, P. J. ; SCOPELLITI, R. ; LAURENCZY, G. ; BELLER, M.** *Angew. Chem. Int. Ed.,* 2010, vol. 49, 9777
- **LIU, J. ; GUO, C. ; ZHANG, Z. ; JIANG, T. ; LIU, H. ; SONG, J. ; FAN, H. ; HAN, B.** *Chemical Communications,* 2010, vol. 46, 5770
- **KROCHER, O. ; KOPPEL, R. A. ; BAIKER, A.** *High Pressure Chemical Engineering,* 1996, vol. 12, 91
- **KAYAKI, Y. ; SUZUKI, T. ; IKARIYA, T.** *Chemistry Letters,* 2001, 1016
- **LIU, F. C. ; ABRAMS, M. B. ; BAKER, R. T. ; TUMAS, W.** *Chemical Communications,* 2001, 433
- **KAYAKI, Y. ; SHIMOKAWATOKO, Y. ; IKARIYA, T.** *Advanced Synthesis & Catalysis,* 2003, vol. 345, 175
- **LIU, F. C. ; ABRAMS, M. B. ; BAKER, R. T. ; TUMAS, W.** *Chemical Communication,s,* 2001, 433
- **KROCHER, O. ; KOPPEL, R. A. ; BAIKER, A.** *Chemical Communications,* 1997, 453
- **U. PLEISS ; R. VOGES.** Synthesis and Applications of Isotopically Labelled Compounds. Wiley-VCH, 2001, vol. 7
- **R. VOGES ; J. R. HEYS ; T. MOENIUS.** Preparation of Compounds Labeled with Tritium and Carbon-14. Wiley-VCH, 2009
- **YONG-JOO KIM ; MAX P. BERNSTEIN ; ANGELA S. GALIANO ROTH ; FLOYD E. ROMESBERG ; PAUL G. WILLIARD ; DAVID J. FULLER ; AIDAN T. HARRISON ; DAVID B. COLLUM.** *J. Org. Chem.,* 1991, vol. 56, 4435-4439
- **R. VOGES ; J. R. HEYS ; T. MOENIUS.** Préparation of Compounds Labeled with Tritium and Carbon-14. Wiley-VCH, 2009
- **CHRISTOPHER J. DINSMORE ; SWATI P. MERCER.** *Organic Letters,* 2004, vol. 6 (17), 2885-2888
- **JOHN T. GROVES ; YOSHIHITO WATANABE.** *J. Am. Chem. SOC.,* 1988, vol. 110, 8443-8452
- **T. A. KOCHINA ; D. V. VRAZHNOV ; E. N. SINOTOVA ; V. V. AVRORIN ; M. YU. KATSAP ; YU. V. MYKHOV.** *Russian Journal of General Chemistry,* 2002, vol. 72 (8), 1222-1224
- **E. A. SHISHIGIN ; V. V. AVRORIN ; T. A. KOCHINA ; E. N. SINOTOVA.** *Russian Journal of General Chemistry,* 2005, vol. 75 (1), 152
- **U. PLEISS ; R. VOGES.** Synthesis and Applications of Isotopically Labelled Compounds, Volume 7. Wiley-VCH, 2001

- **J. Z. HO ; COLL.** *Helvetica Chimica Acta,* 2005, vol. 88, 1040
- **I. V. EKHATO ; S. BONACORSI JR.** *J., Label Compd. Radiopharm,* 2011, vol. 54, 202-205
- **KENNETH K. CHAN ; JAMES A. STAROSCIK.** *Journal of Médicinal Chemistry,* 1977, vol. 20 (4), 598
- **J. Z. HO ; COLL.** *Helvetica Chimica Acta,* 2005, vol. 88, 1040
- **KENNETH K. CHAN ; JAMES A. STAROSCIK.** *Journal of Medicinal Chemistry,* 1977, vol. 20 (4), 598